# EUROPEAN PATENT SPECIFICATION

(11) **EP 3 180 067 B1**
(45) Date of publication and mention of the grant of the patent: **17.02.2021**
(21) Application number: 15831275.1
(22) Date of filing: 13.08.2015
(51) Int. Cl.: A61M 39/16, A61M 39/20

(54) **DISINFECTANT CAPS**
DESINFEKTIONSKAPPEN
CAPUCHONS DÉSINFECTANTS

(30) Priority: 13.08.2014 US 201462036666 P
(43) Date of publication of application: 21.06.2017
(73) Proprietor: Excelsior Medical Corporation, San Clemente, CA 92673 (US)
(72) Inventor: GARDNER, Christopher E., Manalapan, NJ 07726 (US); BANIK, Robert, Long Valley, NJ 07853 (US)
(74) Representative: Lloyd, Patrick Alexander Desmond
(86) International application number: PCT/US2015/045163
(87) International publication number: WO 2016/025775

(56) References cited:
- EP-A1- 2 606 930
- WO-A1-2004/035129
- WO-A1-2010/034470
- US-A1- 2007 112 333
- US-A1- 2007 187 353
- US-A1- 2011 314 619
- US-A1- 2012 031 904
- US-A1- 2012 039 764
- US-A1- 2013 171 030
- US-A1- 2014 101 876
- US-B1- 6 943 035

## Description

### FIELD OF THE DISCLOSURE

The present invention relates to disinfectant caps for medical devices. More specifically, the present invention relates to disinfectant caps for luer access devices that provide direct contact with antiseptic fluid stored therein.

### BACKGROUND

Intravenous (IV) devices are widely used to administer fluids to patients, such as through the use of a catheter inserted into a patient. Usually, the catheter is connected to an injection site, such as a luer access device, which provides fluid communication from a fluid source (e.g., IV bag, syringe, etc.) to the patient. The connectors are frequently separated from each other (e.g., when a patient needs to use the bathroom), which exposes the connectors to the environment, which can result in contamination.

To reduce the risk of contamination, the connectors are usually disinfected between uses. Current procedures include swabbing the connectors with a disinfecting pad, which is prone to human error and not often implemented. Alternatively, antiseptic caps are used to clean and cover the connectors. However, many antiseptic caps require a presoaked absorbent material (e.g., absorbent pad, absorbent sponge, etc.) inserted therein to store and subsequently release the antiseptic fluid onto the connector (or any other medical device). Furthermore, the injection sites typically utilize a male luer thread geometry to facilitate connection of syringes and IV tubing for fluid communication. Existing antiseptic caps utilize the corresponding female luer thread geometry to secure the cap to the injection site. However without the additional tapered luer tip geometry, which secures these types of connections between syringes and injection sites, the antiseptic caps do not securely fit on the injection site, and are prone to falling off inadvertently.

US 2011/314619 discloses a cleaner assembly for cleaning a medical device such as a luer connector. A scrubber is affixed at the bottom and along side surfaces of a cavity and is saturated with antimicrobial fluid. The scrubber has compressible fingers which extend toward a first open end of the cavity and which are engageable by the medical device end portion on insertion so that the fingers scrub the exterior and interior surface of the device end and apply antimicrobial fluid by compression of the scrubber. A second scrubber is affixed to side walls of the cavity adjacent the open end and has flexible projections which extend radially inwardly to scrubs exterior surfaces of the device upon insertion.

US 2012/039764 discloses caps for use in covering and disinfecting a male protrusion portion of a medical connector. The caps can create a seal with the male protrusion to prevent antiseptic from exiting the cap into the fluid pathway.

### SUMMARY

The present invention relates to disinfectant caps and packaging for use with a medical device (e.g., connector, luer access device, etc.). The disinfectant caps apply the antiseptic fluid (e.g., disinfectant) directly onto the surface of the medical device. The disinfectant caps incorporate specific thread geometry to provide a secure fit to threaded access sites.

The invention is defined by the independent claims, to which reference is directed. Preferred features are set out in the dependent claims.

### BRIEF DESCRIPTION OF THE DRAWINGS

The foregoing features of the disclosure will be apparent from the following Detailed Description, taken in connection with the accompanying drawings, in which:
**FIG. 1** is a perspective view of a disinfectant cap with an invertible wall;
**FIG. 2a-2c** illustrate steps for manufacturing the disinfectant cap of **FIG. 1****;**
**FIG. 3a-3b** illustrate steps for applying the disinfectant cap of **FIG. 1** to a luer access device;
**FIG. 4** is a side view of another disinfectant cap with a bulb;
**FIG. 5** is a cross-sectional view of a disinfectant cap with an elastomeric dome cover;
**FIG. 6** is a cross-sectional view of the disinfectant cap of **FIG. 5** engaged with a luer access device;
**FIG. 7** is a perspective view of an elastomeric dome cover with ribs;
**FIG. 8** is a cross-sectional view of the elastomeric dome cover of **FIG. 7****;**
**FIG. 9** is a perspective view of the elastomeric dome cover of **FIG. 7** inverted;
**FIG. 10** is a cross-sectional view of the elastomeric dome cover of **FIG. 7** inverted and attached to a cap body of a disinfectant cap;
**FIG. 11** is a perspective view of an elastomeric dome cover with a helical rib;
**FIG. 12** is a cross-sectional view of the elastomeric dome cover of **FIG. 11** inverted;
**FIG. 13** is a perspective view of a disinfectant cap with an elastomeric dome insert;
**FIG. 14** is a cross-sectional view of the disinfectant cap of **FIG. 13****;**
**FIG. 15** is a cross-sectional view of the disinfectant cap of **FIG. 13** engaged with a luer access device;
**FIG. 16** is a cross-sectional view of a disinfectant cap with a movable plug;
**FIG. 17** is a cross-sectional view of the disinfectant cap of **FIG. 16** engaged with a luer access device;
**FIG. 18** is a cross-sectional view of a disinfectant cap with a threadable insert;
**FIG. 19** is a perspective view of a disinfectant cap with inner variable threads;
**FIG. 20** is a side view of the disinfectant cap of **FIG. 19****;**
**FIG. 21** is a cross-sectional view of the disinfectant cap of **FIG. 19****;**
**FIG. 22** is a perspective view of a disinfectant cap with inner variable thread segments;
**FIG. 23** is a side view of the disinfectant cap of **FIG. 22****;**
**FIG. 24** is a cross-sectional view of the disinfectant cap of **FIG. 22****;**
**FIG. 25** is a top view of the disinfectant cap of **FIG. 22** with a pre-soaked absorbent material inserted therein;
**FIG. 26** is a cross-sectional view of the disinfectant cap of **FIG. 25** with the pre-soaked absorbent material inserted therein;
**FIGS. 27A-27C** are perspective views of a disinfectant cap sealed by a film with a scored area;
**FIGS. 28A-28C** are perspective views of a disinfectant cap with a notch for receiving an attached area of a film attached to a disinfectant cap;
**FIG. 29** is a cross-sectional perspective view of a disinfectant cap with a dome insert sealed in a cap holder;
**FIG. 30** is a cross-sectional side view of the disinfectant cap and cap holder of **FIG. 29****;**
**FIG. 31** is a cross-sectional side view of a disinfectant cap with a dome insert in a cap holder, the disinfectant cap having a bottom opening;
**FIG. 32** is an exploded cross-sectional perspective view of the disinfectant cap and cap holder of **FIG. 31****;**
**FIGS. 33A-33B** are cross-sectional side views illustrating use of the disinfectant cap and cap holder of **FIG. 31****;**
**FIG. 34** is a cross-sectional side view of a disinfectant cap with an integrally formed internal dome barrier in a cap holder, the disinfectant cap having a bottom opening;
**FIG. 35** is an exploded cross-sectional perspective view of the disinfectant cap and cap holder of **FIG. 34****;**
**FIG. 36** is a cross-sectional view of a disinfectant cap with a bottom plug;
**FIG. 37** is a perspective view of a disinfectant cap with a frangible neck;
**FIG. 38** is a cross-sectional perspective view of the disinfectant cap of **FIG. 37****;**
**FIG. 39** is a perspective view of a disinfectant cap with a frangible neck and angled fingers;
**FIG. 40** is a cross-sectional perspective view of the disinfectant cap of **FIG. 39****;** and
**FIGS. 41A-41C** are cross-sectional side views illustrating use of the disinfectant cap of **FIG. 39** and cap holder.

### DETAILED DESCRIPTION

The present disclosure relates to disinfectant caps for disinfecting a luer access device (LAD) or any other medical device. More specifically, the disclosure relates to disinfectant caps with antiseptic fluid (e.g., alcohol, isopropyl alcohol, etc.) to disinfect a surface of an LAD. Many of the disinfectant caps of the present disclosure do not require an absorbent material (e.g., an absorbent sponge or pad). The disinfectant caps described below could be manufactured using any suitable technique (e.g., blow molding, injection molding, etc.), and using one or more of a variety of materials (e.g., polypropylene, polyethylene, etc.). The disinfectant cap could be applied to the medical device regardless of the orientation of the medical device. The features described with respect to a particular embodiment could be used with other embodiments described herein.

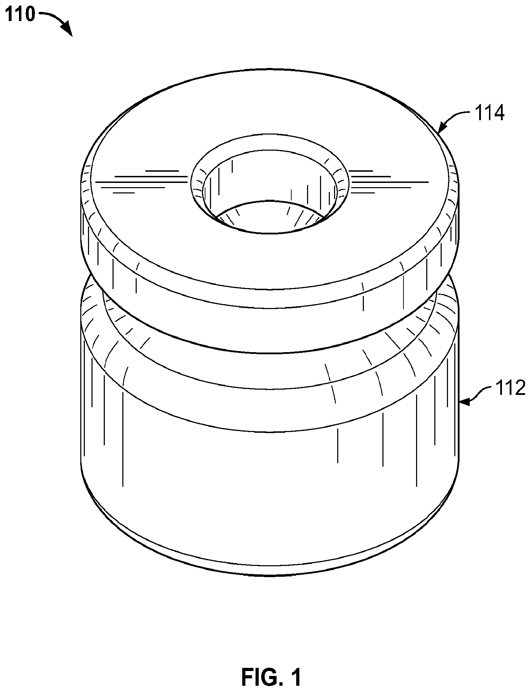

**FIGS. 1-3** show a disinfectant cap **110** with an invertible wall. More specifically, **FIG. 1** is a perspective view of a disinfectant cap **110** with an invertible wall. The disinfectant cap includes a cap body **112** and a cover **114** attached thereto. The cap body **112** could be made out of a rigid or semi-rigid plastic (e.g., polypropylene, polyethylene, etc.) or other suitable material. The cap may be manufactured by a variety of methods including blow molding, injection molding, stamping, vacuum forming, etc.

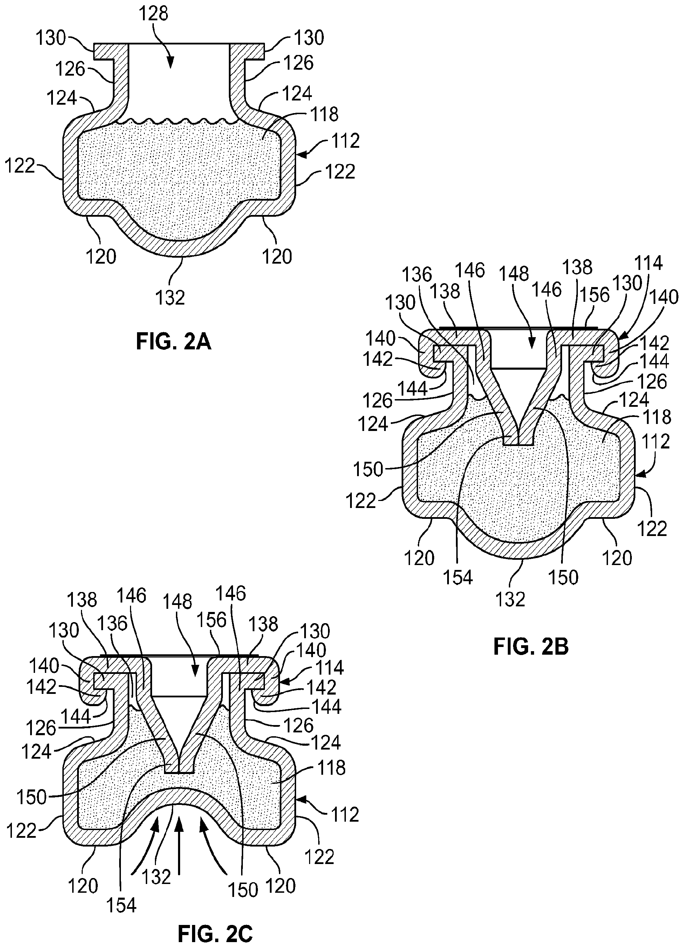

**FIG. 2a-2c** illustrate steps for manufacturing the disinfectant cap of **FIG. 1****.** As shown, in **FIG. 2a****,** the cap body **112** is filled with antiseptic fluid **118** (e.g., alcohol, isopropyl alcohol, ethanol, hydrogen peroxide, povidone iodine, Chlorhexidine Gluconate, triclosan, etc.). The cap body **112** includes a bottom wall **120,** a sidewall **122** extending therefrom, which could include one or more angled segments, such as angled wall **124** (e.g., tapered or horizontal), and a neck **126** extending from sidewall **122,** which all define an interior. The inner circumference of the neck **126** can be smaller than the circumference of the sidewall **122.** The neck **126** defines a top opening **128** providing access to the interior of the cap body **112,** and can include a flange **130** extending (e.g., perpendicularly) from the top of the neck **126.** The bottom wall **120** of the cap body **112** can include an invertible wall **132** which is in a first orientation convex (e.g., outwardly bulging). Although shown as positioned on the bottom wall **120,** the invertible wall **132** could be located anywhere on the cap body **112** (e.g., formed in the sidewall **122).**

In **FIG. 2b****,** a cover **114** is attached to the top of the cap body **112** (e.g., by snapping on, pressing gluing, over-molding, ultrasonically welding, etc.), thereby sealing the antiseptic fluid **118** therein. The cover **114** could be made of a hard plastic. Once attached and sealed, the cover **114** and the cap body **112** define an interior having a first volume, and the antiseptic fluid **118** and gas **136** (e.g., air or atmosphere), if any, are stored therein at a first pressure (e.g., atmospheric pressure).

The cover **114** can include a planar circular top portion **138** with a depending rim **140** extending downwardly from a peripheral edge of the planar circular top portion **138.** The depending rim **140** can include a lip **142** extending (e.g., perpendicularly) from a bottom thereof. The lip **142** could include an annular rounded edge **144** (or taper) to facilitate application of the cover **114** to the cap body 112. The lip **142** of the rim **140** engages the flange **130** of the neck **126** of the cap body **112** to secure the cover **114** to the cap body **112.** However, any form of attachment could be used (e.g., friction fit, adhesion, welding, overmolding, etc.).

The cover **114** can include an inner wall **146** extending downwardly from the top portion **138** of the cover **114** and defining a central aperture **148.** The inner wall **146** can include a portion of reducing size **150** (e.g., tapered, conical, etc.) extending downwardly to a frangible tip **154** that extends into the interior of the cap body **112.** The inner walls **146** can be made of a hard (e.g., rigid, semi-rigid, etc.) plastic and the frangible tip **154** forms a breakable nozzle. Alternatively, the inner walls **146** can be made of a soft material (e.g., rubber, low durometer material, silicone or thermoplastic) and the tip **154** can be slit or manufactured with a small opening (e.g., hole or slit). The inner walls **146** and the tapered portion **150** are sized and shaped to engage a medical implement that requires disinfecting, such as a luer access device (not shown). When the cover **114** is attached to the cap body **112,** the frangible tip **154** may be submerged in the antiseptic fluid **118** contained in the cap body **112.** A removable film **156** (e.g., paper peel, peelable sterile barrier, foil lidstock, etc.) could be adhered (or otherwise attached) to the top surface of the cover **114,** such as over the central aperture **148.**

It is noted that the cap body **112** does not require a neck **126** (e.g., the sidewall **122** defines a top opening of the cap body **112),** and that any shape or type of cap body **112** and/or cover **114** could be used. The cover **114** could include threads (or other mating feature such as protrusions, recesses, and/or snap fits) molded into the inner walls **146** (such as in the tapered portion **150)** to mate with and retain the LAD. Alternatively, the inner walls **146** could be sized to form an interference fit with the LAD. The inner walls **146** could be made of a material of sufficient softness to allow threads on the LAD to penetrate into the surface of the inner walls **146** so as to create mating female threads on the smooth surface.

In **FIG. 2c****,** after the disinfectant cap **110** is filled and sealed, pressure can be exerted (e.g., by a user, by a machine) upon the invertible wall **132** of the bottom wall **120** until the invertible wall **132** deforms inwardly to a second concave orientation. The inward deformation (e.g., deflection) of the invertible wall **132** decreases the volume of the interior defined by the cover **114** and the cap body **112,** thereby increasing the pressure within the disinfectant cap **110** (e.g., so that the pressure is greater than atmospheric pressure).

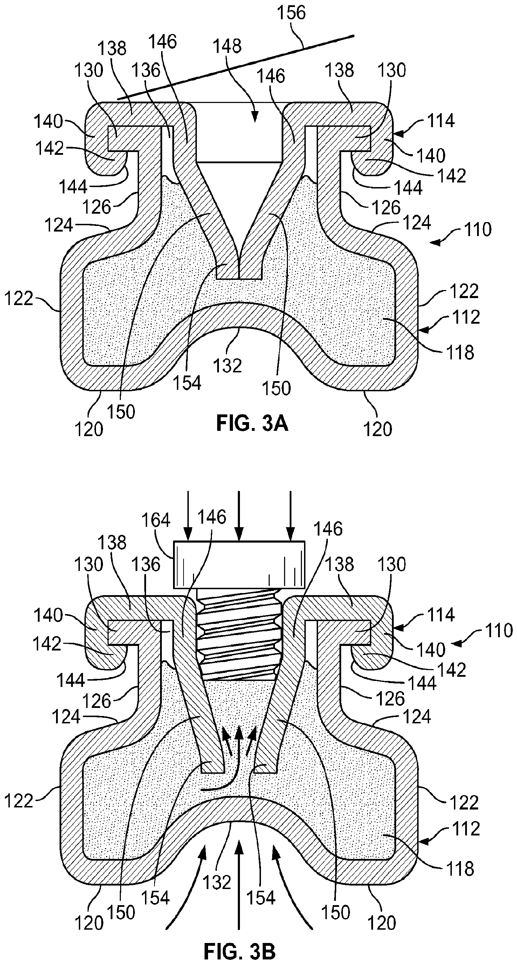

**FIG. 3a-3b** illustrate steps for applying the disinfectant cap **110** of **FIG. 1** to a luer access device. In **FIG. 3a****,** a user removes the film **156** adhered to the top of the cover **114,** thereby providing access to the central aperture **148** defined by the cover **114.** In **FIG. 3b****,** the disinfectant cap **110** is applied to an LAD **164** (or other medical device) by inserting (e.g., pushing, threading, etc.) the LAD **164** into the central aperture **148.** The tapered portion **150** of the cover **114** may be smaller in size (e.g., diameter) than the LAD **164.** In this way, when the LAD **164** is inserted into the aperture **148,** the frangible tip **154** of the tapered portion **150** breaks, so that the LAD **164** is wedged between the tapered portion **150** and fully engaged with the disinfectant cap **110.** Once the frangible tip **154** breaks the pressurized antiseptic fluid **118** sprays out onto the surface of the LAD **164** (because the pressure within the disinfectant cap **110** is greater than atmospheric pressure) and fills the area around the LAD **164,** thereby disinfecting the LAD **164.** Alternatively, the invertible wall **132** could be deformed after application of the disinfectant cap **110** to the LAD **164.** Deforming the invertible wall after application of the disinfectant cap to the LAD may be preferable if the frangible tip **154** is replaced by a slit or other small opening. In this configuration the disinfectant may not be forced through the opening upon removal of the film. Rather, upon insertion of the LAD, the opening at the tip **154** becomes larger in size to allow greater fluid flow from the cavity **118** onto the LAD. The initial size of the slit or opening at the tip **154** can be sized to minimize the amount of disinfectant that might leak through the tip **154** if the cap **120** is positioned upside down prior to insertion of the LAD. For example, a small hole or slit may be prone to contain the disinfectant as a liquid tends not to flow through a small hole unless pressurized (e.g., due to liquid surface tension).

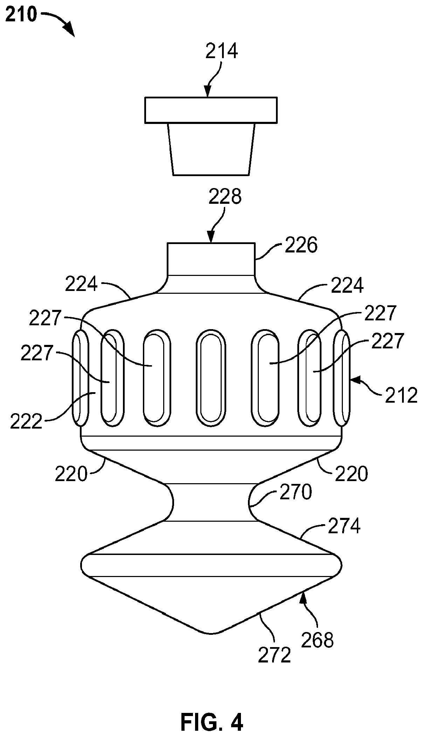

**FIG. 4** is a side view of another disinfectant cap **210** with a bulb. More specifically, the disinfectant cap **210** includes a cap body **212** and a cover **214.** The cap body **212** includes a bottom wall **220,** a sidewall **222** which could include one or more angled segments such as angled wall **224,** and a neck **226,** all of which define an interior, as described above. The outer surface of the sidewall **222** could include a plurality of annularly spaced vertical ribs **266** oriented along the long central axis of the disinfectant cap **210.** The vertical ribs **227** facilitate gripping and twisting of the disinfectant cap **210** by a user.

The inner circumference of the neck **226** is smaller than the circumference of the sidewall **222.** The neck **226** defines a top opening **228** providing access to the interior of the cap body **212.** A cover **214** (e.g., breakable seal insert) is inserted into the neck **226** to seal the interior of the disinfectant cap **210.**

The bottom wall **220** of the cap body **212** includes a bulb **268** integrally formed with (or attached to) the cap body by a stem **270.** The circumference of the bulb **268** could be sized similarly to, or smaller than, the circumference of the cap body **212.** The bulb **268** defines an interior, which is in fluid communication with the interior of the cap body **212** via a channel within the stem **270.**

The bottom surface **272** (and/or top surface **274)** of the bulb **268** could be invertible to decrease the volume of the disinfectant cap **210** and increase the pressure of the antiseptic fluid and any gas contained therein. The bulb **268** could be deformed before or after engagement with an LAD, such as by squeezing the bulb **268** to direct disinfecting fluid through the stem **270** and onto an LAD or other medical device. The bulb **268** can be designed such that it retains its deformed shape to maintain internal pressure. For example, the bottom surface **272** (e.g., bottom wall) of the bulb **268** could be designed with a slight outward bow so that as pressure is exerted the bottom surface **272** eventually reaches a point of inflection where it flexes and inverts, and then remains nested in the top surface **274** (e.g., similar to a locking bellow).

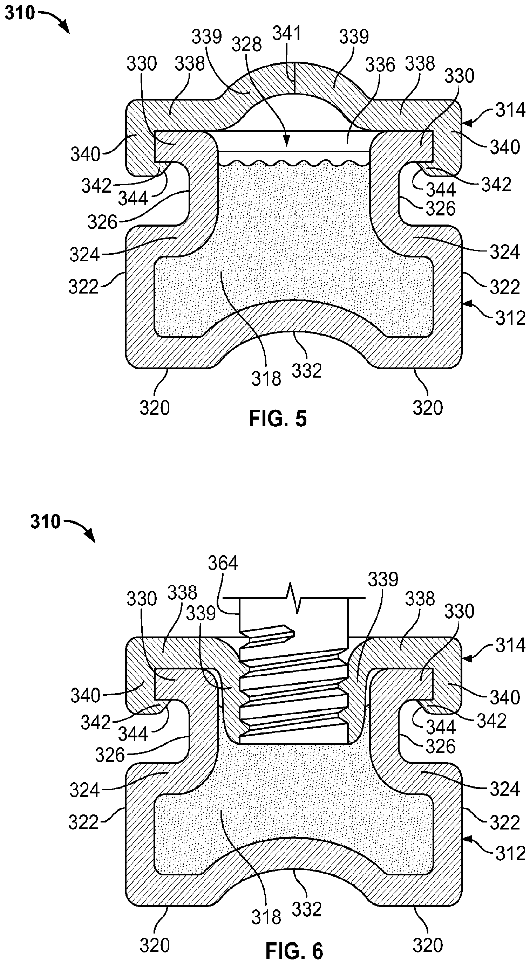

**FIGS. 5-12** show disinfectant caps with domed covers. More specifically, **FIG. 5** is a cross-sectional view of a disinfectant cap **310** with an elastomeric dome cover **314.** The disinfectant cap **310** of **FIGS. 5-12** includes a cap body **312,** which includes a bottom wall **320,** a sidewall **322** which could include one or more angled segments such as angled wall **324,** and a neck **326,** all of which define an interior, as described above. The neck **326** defines a top opening **328** providing access to the interior of the cap body **312,** and includes an outwardly extending flange **330** extending from the top of the neck **326.** The disinfectant cap **310** shown in **FIG. 5** is fully assembled and manufactured such that the bottom wall **320** of the cap body **312** includes an invertible wall **332,** but the invertible wall is not required and the bottom wall **320** could be of any shape and contour.

The elastomeric dome cover **314** includes a generally planar outer periphery **338** with a centrally located dome portion **339.** A depending rim **340** extends downwardly from the outer periphery **338** a peripheral edge of the outer periphery **338.** The rim **340** include a lip **342** extending from a bottom of the rim **340.** As described above, the lip **342** of the rim **340** engages the flange **330** of the neck **326** of the cap body **312** to secure the elastomeric dome cover **314** to the cap body **312.** However, any form of attachment could be used (e.g., friction fit, adhesion, etc.).

The elastomeric dome cover **314** includes a dome portion **339** (e.g., hemispherical wall) having one or more partial slits **341** formed through (or near) the apex thereof. The elastomeric dome cover **314** is attached to the top of the cap body **312,** thereby retaining antiseptic fluid **318** and gas **336,** if any, therein. Alternatively, the disinfectant cap **310** could be filled with antiseptic fluid **318** through the partial slits **341.** Further, instead of partial slits **341,** the elastomeric dome cover **314** could have a weakened area made by other means (e.g., a thin wall that separates when pressure is applied to the cover or when the cover is inverted). Instead of partial slits **341,** the elastomeric dome cover **314** could have a small hole which retains the disinfectant in the cavity through the surface tension of the liquid. The elastomeric dome cover **314** (e.g., and partial slits **341** or small hole) could be sealed with a lidstock.

The elastomeric dome cover **314** and the cap body **312** define an interior having a first volume and the antiseptic fluid **318** and gas **336** (e.g., air or atmosphere), if any, is stored therein at a first pressure (e.g., atmospheric pressure). A removable film could be applied (e.g., adhered) to the top surface of the elastomeric dome cover **314.**

**FIG. 6** is a cross-sectional view of the disinfectant cap **310** of **FIG. 5** engaged with a luer access device **364.** To apply the disinfectant cap **310** to an LAD **364,** a user removes the film (if any) from the top surface of the elastomeric dome cover **314.** The disinfectant cap **310** is then applied to the LAD **364** (or other medical device) so that the LAD **364** contacts and then begins to compress and deform the elastomeric dome cover **314** such that the elastomeric dome cover **314** deforms inwardly, which decreases the volume of the interior of the disinfectant cap **310** and increases the pressure of the antiseptic fluid **318** and any gas **336** therein. The elastomeric dome cover **314** continues to deform and invert until the slit **341** at the apex of the dome portion **339** opens and pressurized antiseptic fluid **318** sprays on the surface of the LAD **364.** The LAD **364** continues to engage the disinfectant cap **310** until it is frictionally secured to the inwardly deformed dome portion **339** of the cover **314** (e.g., the LAD **364** enters the inverted dome cover **314)** and/or through the opened slits **341** of the dome cover **314.** The elastomeric dome cover **314** stretches around the LAD **364,** thereby securing itself to the LAD **364** (which eliminates the need for threads) and sealing the antiseptic fluid **318** between the dome cover **314** and the LAD **364.** Alternately, the inner surface of the cap body **312** (e.g., sidewall **322,** neck **326,** etc.) could incorporate threads, snaps, detents, or other engagement features to more securely attach the cap **310** to the LAD after the elastomeric dome cover **314** has been penetrated.

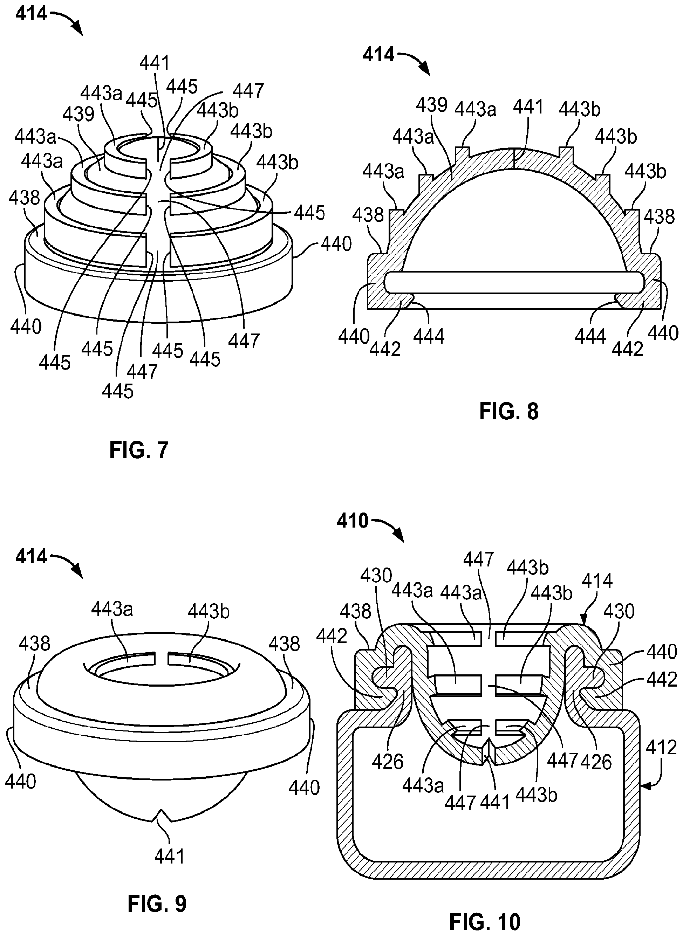

**FIGS. 7-10** show an elastomeric dome cover **414** with ribs **443** that extend around the surface of the cover **414.** The elastomeric dome cover **414** includes a planar circular top edge portion **438** with a centrally located dome **439.** A rim **440** extends downwardly from a peripheral edge of the planar circular top edge portion **438.** The rim **440** include an inwardly extending lip **442** extending from a bottom of the rim **440.** The dome **439** has one or more partial slits **441** (and/or weakened area) formed through (or near) the apex thereof. The dome cover **414** can include concentric, outwardly extending, ribs **443** (e.g., circumferential rings). Although ribs **443** are described, other types of outwardly extending protrusions could be used.

The ribs **443** could extend vertically or horizontally or radially from the dome **439.** The ribs **443** are non-continuous with aligned opposite ends **445** such that each rib **443** is composed of two rib segments (e.g., a first rib segment **443a** and a second rib segment **443b).** The breaks **447** between the rib segments **443** facilitate inversion of the elastomeric dome cover **414** by allowing the dome **439** to bend/flex. Although two rib segments **443** are shown, any number of rib segments **443** could be used, or a single non-continuous rib (e.g., with only one break).

**FIG.** 8 is a cross-sectional view of the elastomeric dome cover **414** of **FIG.** 7. As shown, the rim **440** includes a lip **442** extending from a bottom of the rim **440.** An annular groove could be formed in the rim **440** to receive the cap body **412.** The lip **442** could include an annular taper **444** to facilitate application of the elastomeric dome cover **414** to the cap body **412.**

**FIG. 9** is a perspective view of the elastomeric dome cover **412** of **FIG. 7** inverted. As shown, the slit **441** of the elastomeric dome cover **412** is naturally forced open by inversion of the elastomeric dome cover **412.**

**FIG. 10** is a cross-sectional view of the elastomeric dome cover **414** of **FIG. 7** inverted and attached to a cap body **412** of a disinfectant cap **410.** The lip **442** of the rim **440** engages the flange **430** of the neck **426** of the cap body **412** to secure the elastomeric dome cover **414** to the cap body **412.** When the elastomeric dome cover **414** is inverted the ribs **443** extend inwardly to facilitate engagement with the LAD (not shown).

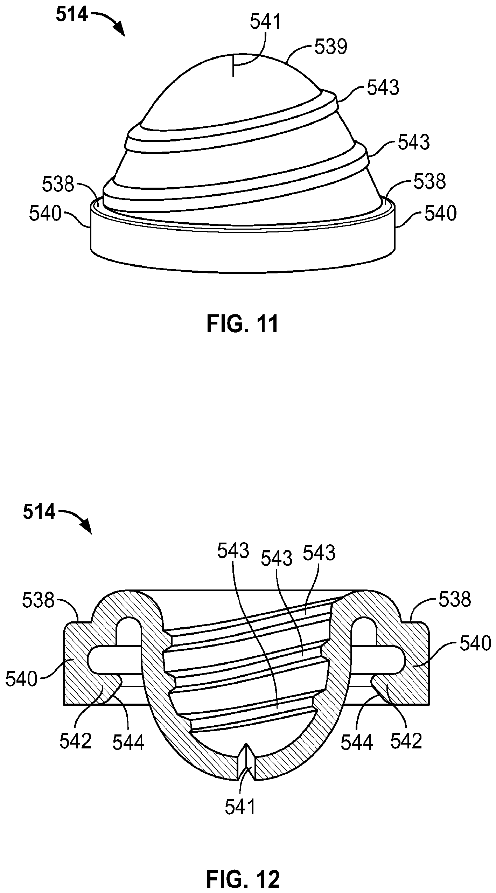

**FIGS. 11-12** show another elastomeric dome cover **514** with a helical rib **543.** More specifically, **FIG. 11** is a perspective view of an elastomeric dome cover **514** with an outwardly extending helical rib **543** (e.g., spiral rib). The helical rib **543** could be continuous or non-continuous (the helical rib **543** could include one or more breaks). The elastomeric dome cover **514,** as previously described, includes a planar circular top edge portion **538** with a centrally located dome **539.** A depending rim **540** extends downwardly from a peripheral edge of the planar circular top edge portion **538.** The dome **539** has one or more partial slits **541** (and/or weakened area) formed through (or near) the apex thereof.

**FIG. 12** is a cross-sectional view of the elastomeric dome cover **514** of **FIG. 11** inverted. As shown, the rim **540** includes a lip **542** extending from a bottom of the rim **540.** An annular groove could be formed in the rim **540** to receive the cap body **512.** The lip **452** includes an annular taper **544** to facilitate application of the elastomeric dome cover **514** to the cap body. As shown, the slit **541** of the elastomeric dome cover **514** is forced open when inverted. When the elastomeric dome cover **514** is inverted the helical rib **543** extends inwardly to facilitate engagement with the LAD (not shown). In this way, the helical rib **543** can form threads to engage the threads of the LAD.

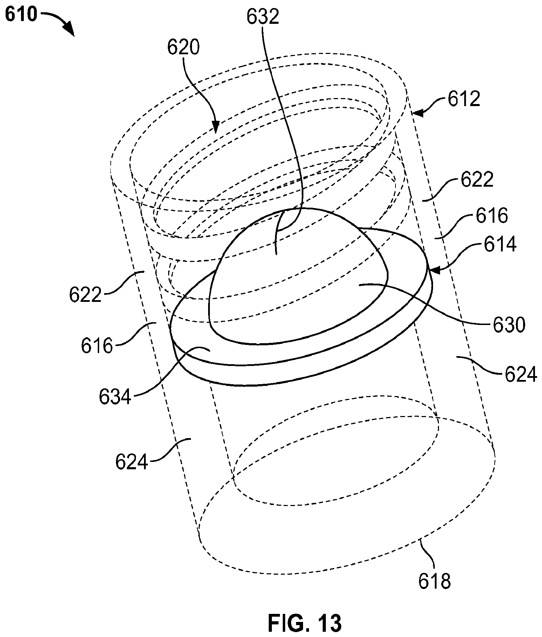

**FIGS. 13-15** show a disinfectant cap **610** with an elastomeric dome insert **614.** More specifically, **FIG. 13** is a perspective view of a dome insert **614** in a disinfectant cap **610** (shown in dashed lines). The elastomeric dome insert **614** is positioned within the interior of the cap body **612.** The elastomeric dome insert **614** includes a dome **630** (e.g., hemispherical wall) having one or more slits **632** (and/or weakened areas) formed through (or substantially near) the apex thereof. The elastomeric dome insert **614** further includes an annular disc **634** extending outwardly from the base of the dome **630.**

As shown, the disinfectant cap **610** includes a cap body **612** with a sidewall **616** and a bottom wall **618** defining an interior. The top of the sidewall **616** defines a top opening **620.** Although not shown, a removable film could be provided over the top opening **620.** The sidewall **616** includes an upper portion **622** and a lower portion **624,** with a shoulder (e.g., ledge) **626** therebetween, such that the wall of the upper portion **622** has a smaller thickness than the wall of the lower portion **624.** The annular disc **634** rests on the shoulder (e.g., ledge) **626** of the cap body **612** when positioned therein.

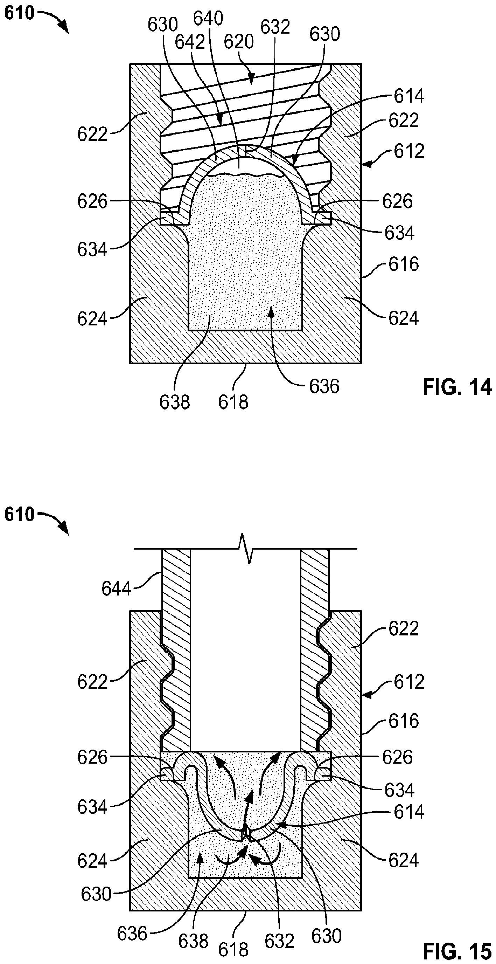

**FIG. 14** is a cross-sectional view of the disinfectant cap **610** of **FIG. 13****.** The lower portion **624** of the sidewall **616** of the cap body **612** and the bottom surface of the elastomeric dome insert **614** define a first chamber **636** for containing antiseptic fluid **638,** and any gas or air **640,** therein at a first volume and a first pressure. The antiseptic fluid **638** could fill the interior of the disinfectant cap such that the fluid level rises above the ledge **626** of the cap body **612.**

The upper portion **622** of the sidewall **616** of the cap body **612** and the top surface of the elastomeric dome insert **614** define a second chamber **642** for receiving and engaging an LAD (not shown). The inner surface of the upper portion **622** of the sidewall **616** could be threaded and/or elastically deformable to engage the LAD. Alternate engagement features or mechanisms could be employed such as press fits, snap fits, grooves, recesses, etc.

**FIG. 15** is a cross-sectional view of the disinfectant cap of **FIG. 13** engaged with a luer access device **644.** As shown, the disinfectant cap **610** threadably engages the LAD **644,** until the LAD **644** makes contact with the elastomeric dome insert **614.** As the LAD **644** continues to engage the disinfectant cap **610,** the LAD **644** begins to deform the elastomeric dome insert **614** such that it deforms inwardly, which decreases the volume of the first chamber **638** and increases the pressure of the antiseptic fluid **638** and gas **640,** if any, therein. The elastomeric dome insert **614** continues to deform until the slit **632** at the apex of the dome **630** opens and pressurized antiseptic fluid **638** sprays onto the surface of the LAD **644.** In lieu of a slit **632,** the elastomeric dome insert **614** could employ a small hole that allows the disinfectant liquid to pass through it when the dome is depressed by the LAD. The hole could be sized to retain the disinfectant liquid within the first chamber **636** (e.g., due to the liquid surface tension). The elastomeric dome insert **614** can be secured to the cap **612** by any means including snap fit, adhesion, bonding, ultrasonic welding, press fit, etc. Alternately, the cap **612** and elastomeric dome insert **614** can be made of a single component (e.g., integrally formed together).

It may be desirable to design the cap to allow evaporation of the disinfectant after application of the cap to the LAD. A significant residual pool of disinfectant can remain on the access site and increase the risk of infusing the disinfectant into the patient (in the example of disinfecting an LAD). Without any absorbent material present to remove residual disinfectant, it may be beneficial to allow excess disinfectant to evaporate. This can be accomplished by designing channels, slits, holes, gaps in the threading, or other means through which the disinfectant can evaporate to the external atmosphere.

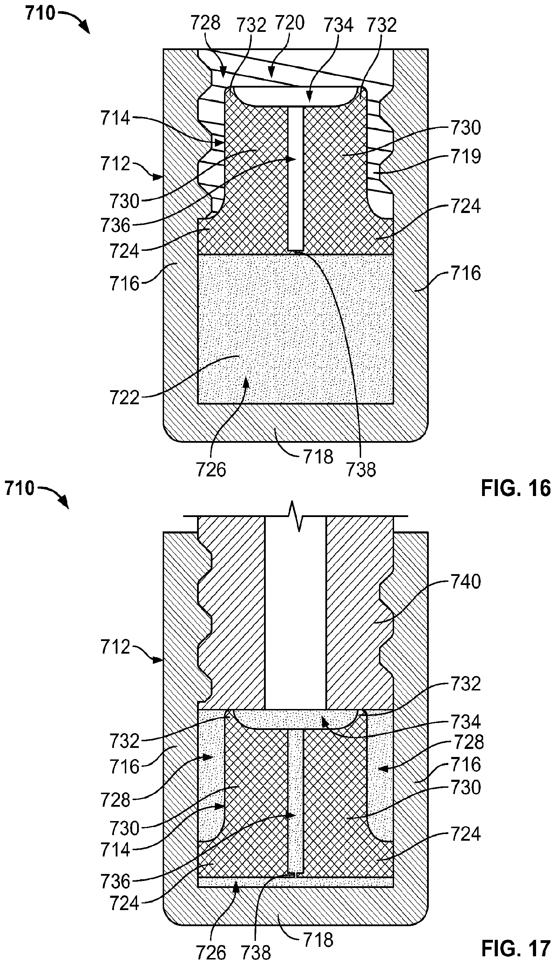

**FIGS. 16-17** show a disinfectant cap **710** with a movable plug **714.** More specifically, **FIG. 16** is a cross-sectional view of a disinfectant cap **710** with a movable plug **714.** As shown the disinfectant cap **710** includes a cap body **712** with a sidewall **716** and a bottom wall **718** defining an interior. The top of the sidewall **716** defines a top opening **720** which received the LAD (not shown). Although not shown, a removable film could be provided over the top opening **720.** The inner surface of the sidewall **716** could be threaded **719** and/or deformable to engage the LAD.

Antiseptic fluid **722** is inserted into and contained within the cap body **712.** A movable plug **714** (e.g., wall, divider, etc.) is positioned within the interior of the cap body **712** at or above the antiseptic fluid **722.** The movable plug **714** includes a cylindrical bottom end **724** which has approximately the same size as the interior diameter of the cap body **712,** and could form a friction fit with the cap body **712,** or an o-ring could be added around the periphery of the bottom end **724** to prevent the antiseptic fluid **722** from seeping past the movable plug **714.** The bottom surface of the bottom end **724** of the movable plug **714,** the sidewalls **716,** and the bottom wall **718** define a first chamber **726.** The top surface of the movable plug **714** and the sidewalls **716** of the cap body **712** define a second chamber **728.**

The movable plug further comprises a recessed section **730** extending from the bottom end **724.** The upper portion of the movable plug **714** includes an annular rim **732,** which defines a recess **734** therein and the annular rim **732** could be continuous or non-continuous (e.g., could include one or more breaks), and/or have one or more scallops in a top surface thereof. The recessed section **730** has a diameter that is smaller than that of the bottom end **724,** to facilitate movement of the plug **714** does not interfere with the threads. Alternatively, instead of the recessed section **730,** the rim **732** could simply extend from the top of the bottom end **724** (so that the entire plug **714** has one continuous sidewall of a substantially consistent diameter).

The recessed section **730** defines a vertical channel **736** extending from a top of the recessed section **730** through (or nearly through) a bottom of the bottom end **724.** At the bottom of the channel **736** there could be an access point **738** (e.g., small hole, fluid seal, valve, or a frangible element built into (or attached to) the bottom end **724** of the plug **714** that prevents fluid from seeping into the channel **736.** For example, the movable plug **714** may be made of a rigid material (e.g., polypropylene, polyethylene, etc.) with an access point **738** being a weaker area molded into the bottom end **724** of the plug **714,** or the movable plug **714** could be an elastomer (e.g., silicone) with the access point **738** being a valve (e.g., check valve, duck-bill valve, thin slit valve, etc.) or small hole molded into the bottom end **724** of the plug **714.**

**FIG. 17** is a cross-sectional view of the disinfectant cap **710** of **FIG. 16** engaged with a luer access device **740.** As shown, the disinfectant cap **710** threadably engages (e.g., is placed onto) the LAD **740,** until the LAD **740** makes contact with the annular rim **732** of the movable plug **714.** As the LAD **740** continues to engage the disinfectant cap **710,** the LAD **740** forces the movable plug **714** to translate into the interior of the cap body **712,** which increases the pressure of the antiseptic fluid **722.** The increased pressure forces the antiseptic fluid **722** through the access point **738** (e.g., breaks the frangible element, overcomes the retaining force of the valve, overcomes the liquid surface tension to pass through the hole, cracks the valve, etc.), so that antiseptic fluid **722** is then forced upward through the vertical channel **736** (e.g., the fluid **722** is displaced by advancing the plug **714** into the disinfectant cap **710).** Once at the top of the vertical channel **736,** the antiseptic fluid **722** then flows onto the surface of the LAD **740** and/or pools in the recess **734** defined by the rim **732** until overflowing into the space defined by the exterior surface of the recessed section **730** and the interior surface of the cap body **712.** Once sufficiently engaged, the pooled antiseptic fluid **722** continuously contacts the surface of the LAD **740.** In this way the antiseptic fluid **722** moves from the first chamber **726** to the second chamber **728,** which submerges the plug **714.**

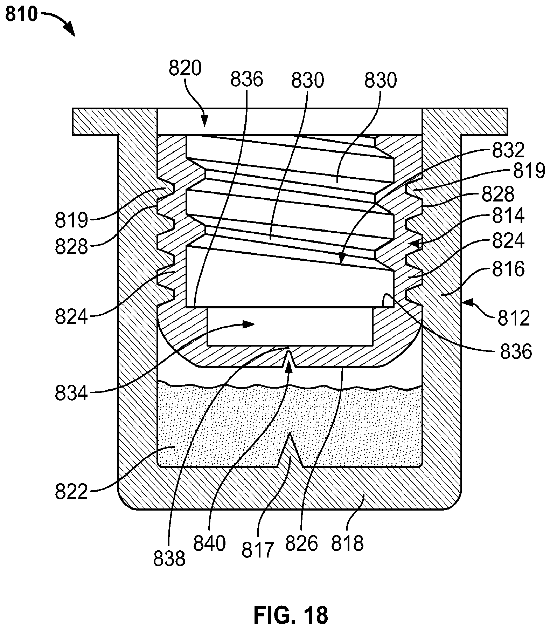

**FIG. 18** is a cross-sectional view of a disinfectant cap **810** with a threadable insert **814** (e.g., movable plug). As shown the disinfectant cap **810** includes a cap body **812** with a sidewall **816** and a bottom wall **818** defining an interior. The top of the sidewall **816** defines a top opening **820** which receives the LAD (not shown). Although not shown, a removable film could be provided over the top opening **820.** The inner surface of the sidewall **816** could be threaded **819** and/or deformable to engage the LAD. A conical spike **817** (e.g., sharp protrusion) extends inwardly from an (approximate) center of an interior surface of the bottom wall **818.**

Antiseptic fluid **822** is inserted into and contained within the cap body **812.** A threadable insert **814** is positioned within the interior of the cap body **812** at, or above, the antiseptic fluid **822** (thereby retaining the antiseptic fluid **822** in the cap body **812).** The threadable insert **814** includes a sidewall **824** and a bottom wall **826** defining an interior. The threadable insert **814** includes outer threads **828** defined by an exterior surface of the sidewall **824,** which engage the threads **819** of the sidewall **816** of the cap body **812,** and could provide a seal therebetween. The threadable insert **814** also includes inner threads **830** defined by an interior surface of the sidewall **824,** which engage the threads of the LAD (not shown). Alternatively, the threadable insert **814** could utilize a friction fit (instead of the outer threads **828** and/or inner threads **830).**

The sidewall **824** of the threadable insert **814** includes an upper portion **832** and a lower portion **834,** where the sidewall **824** of the upper portion **832** is thinner than that of the lower portion **834,** thereby defining an annular ledge **836.** The bottom wall **826** includes an access point **838** (e.g., frangible element and/or fluid seal) in the center of the bottom wall **818.** Further, the access point **838** could define a conical recess **840** in the exterior surface thereof, where the conical recess **840** is positioned directly in line with (and could be correspondingly shaped to) the spike **817.**

To apply the disinfectant cap **810** to the LAD, the disinfectant cap **810** is threaded onto the LAD, so that the threadable insert **814** moves relative to the LAD. Once the LAD bottoms out and contacts the ledge **836** of the threadable insert **814** (e.g., the LAD cannot engage the threadable insert any more), the threadable insert **814** stops moving relative to the LAD, and instead moves relative to the cap body **812.** The disinfectant cap **810** is continued to be threaded and move laterally into the interior of the cap body **812,** pressure could build by the decreased volume between the bottom wall **826** of the threadable insert **814** and the bottom wall **818** of the cap body **812.** The cap **810** continues to move inwardly until the spike **817** of the cap body **812** punctures the access point **838** of the bottom wall **826** of the threadable insert **814.** Once punctured, the pressurized antiseptic fluid **822** flows onto the surface of the LAD and pools in the lower portion **834** of the threadable insert **814** so that the antiseptic fluid **822** makes continuous contact with the LAD. The conical recess **840** of the bottom wall **826** of the threadable insert **814** delays the puncturing of the access point **838,** which further decreases the volume (increasing the pressure) of the antiseptic fluid **822** and gas, if any.

Notably, the spike **817** of the disinfectant cap of **FIG. 18** is not required and could be replaced with a valve or frangible element in the bottom wall of the threadable insert **814** (which breaks upon sufficient pressure), as described with respect to **FIGS. 16-17****.** In this respect, the disinfectant cap of **FIG. 16-17** could utilize the spike **817** discussed with the disinfectant cap **810** of **FIG. 18****.** Alternately, the threadable insert **814** could have a hole in the bottom wall **826,** which is sized to prevent the disinfectant fluid **822** from flowing therethrough (e.g., due to liquid surface tension), until the disinfectant fluid **822** is pressurized by threading of the threadable insert **814** into the disinfectant cap **810.**

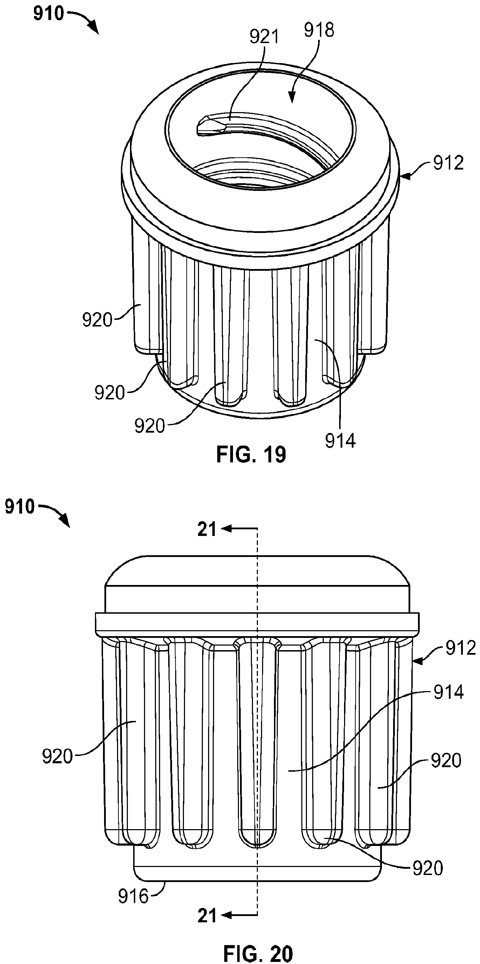

**FIGS. 19-21** are views of a disinfectant cap **910** with inner variable threads **921.** More specifically, **FIG. 19** is a perspective view of a disinfectant cap **910** with inner variable threads **921,** and **FIG. 20** is a side view of the disinfectant cap of **FIG. 19****.** Although variable threads are discussed with respect to a disinfectant cap, the variable threads could be used for any threaded medical device (e.g., any threaded luer access device). Further, the variable threads shown could be used with any of the embodiments discussed above.

The disinfectant cap **910** includes a cap body **912** with a sidewall **914** and a bottom wall **916** defining an interior. The top of the sidewall **914** defines a top opening **918.** Although not shown, a removable film could be provided over the top opening **918.** The outer surface of the sidewall **916** could include a plurality of annularly spaced vertical ribs **920** oriented along the axis of the disinfectant cap **910.** The vertical ribs **920** facilitate gripping and twisting of the disinfectant cap **910** by a user. The inner surface of the sidewall **914** includes one or more variable threads **921.** The disinfectant cap **910** can be stored in a second component, which can serve as an applicator and/or as packaging. The vertical ribs **920** can provide rotational lock between the disinfectant cap **910** and the second component (e.g., applicator). Lidstock can be applied to the second component (e.g., applicator) to provide a fully sterile packaging for the disinfectant cap **910.** In applications where the cap **910** is stored within a second component, the lidstock can be adhered to both the cap **910** and the second component to provide two distinct chambers. Alternately, the cap can **910** be detached from the lidstock such that it is open within the chamber that is formed by the second component and the lidstock.

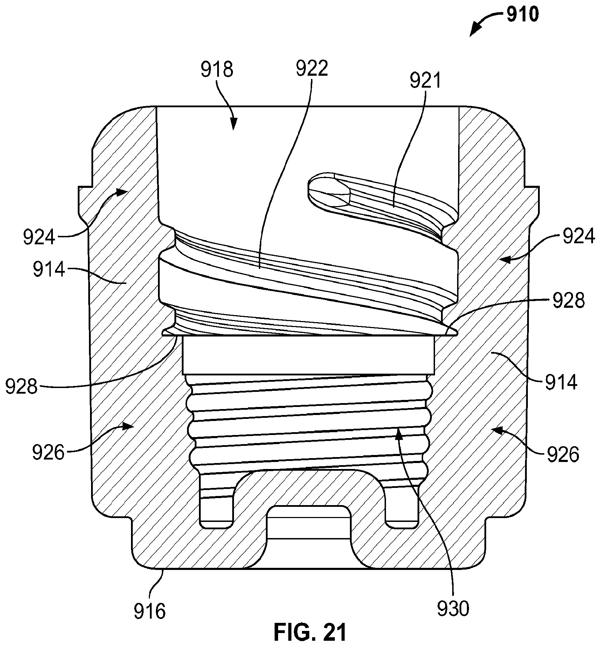

**FIG. 21** is a cross-sectional view of the disinfectant cap **910** of **FIG. 19****.** The sidewall **914** includes an upper portion **924** and a lower portion **926,** where the wall of the upper portion **924** is thinner than that of the lower portion **926,** thereby defining an annular ledge **928.**

The inner surface of the upper portion **924** of the disinfectant cap **910** includes variable pitch threads **921.** For the disinfectant cap **910,** the threads **921** (e.g., double threads) start out at a constant pitch (e.g., starting pitch), such as for an approximate 1/4-3/4 of a turn, and then change pitch thereafter. The starting pitch is that of a standard luer lock design to assist the user with applying the disinfectant cap **910** to an LAD (e.g., relatively low torque). By varying the pitch (e.g., by reducing or increasing the pitch), as the disinfectant cap **910** is threaded onto the LAD (or other medical device), the consistent standard threads of the medical device wedge with the mismatched variable pitch threads **921** of the disinfectant cap **910.**

Wedging the threads together in this fashion provides a more secured connection (e.g., from unthreading or loosening from incidental handling or contact) because a higher removal torque is needed to disengage the disinfectant cap **910** from the LAD. This design allows for engagement of the disinfectant cap **910** onto a threaded medical device (e.g., luer threads), with no other engagement mechanism (e.g., luer taper). Without a luer taper, the proposed reducing pitch threads **921** provide a secure fit than traditional luer threads alone. To create and maintain the wedge, the disinfectant cap **910** could be made of a variety of materials, such as a hard plastic (e.g., high-density polyethylene (HDPE)). LADs usually have ACME profiled threads or modified ACME profiled threads (e.g., stub ACME threads), which are known for power transmission applications due to the flat sides which distribute stress well over the faces of the thread. As a result, the ACME type threads transmit high torques while minimizing stress, which translates to better wedging action and higher removal torques.

Further, the inner diameter of the side wall **914** of the disinfectant cap **910** (e.g., major and/or minor inner diameters of the threads) can reduce in diameter further into the disinfectant cap **910.** This can provide an interference fit with the outer diameter (e.g., the threads) on the medical implement. Additionally, the start of the threads **912** could be offset from the top opening **918.** This could require partial insertion of the luer access device into the disinfectant cap **910,** which provides alignment of the disinfectant cap **910** and luer access device before threading and facilitates threading of the disinfectant cap **910** onto the luer access device.

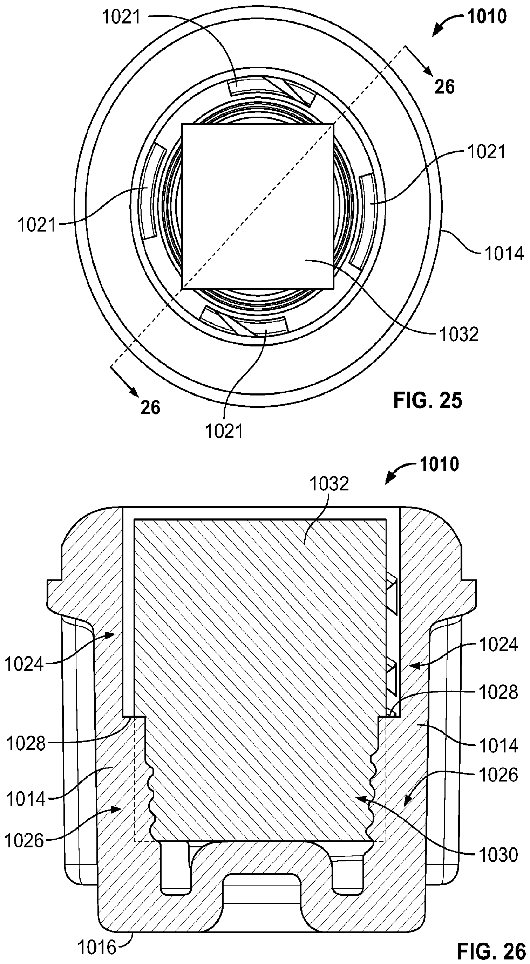

The lower portion **926** of the inner surface of the sidewall **914** includes retaining rings or threads **930,** which is discussed in more detail below with respect to **FIGS. 25-26****.**

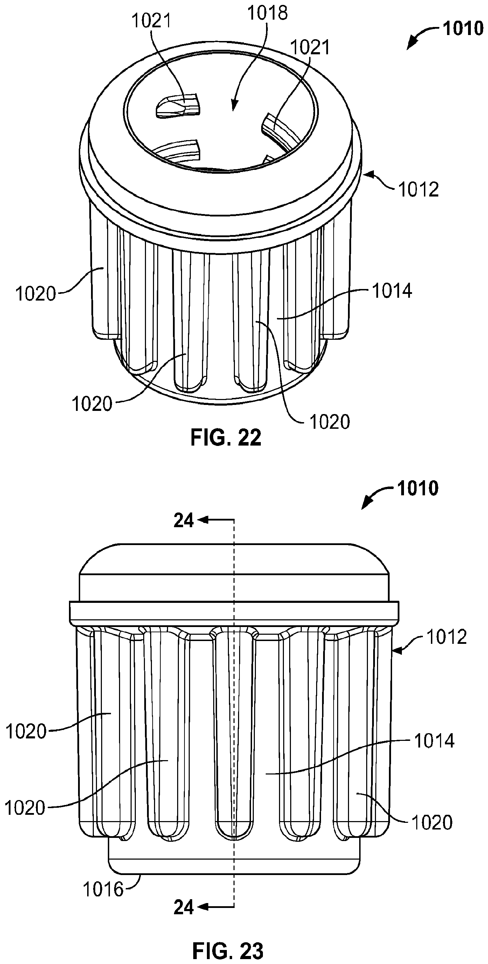

**FIGS. 22-26** are views of a disinfectant cap **1010** with inner variable thread segments **1021.** More specifically, **FIG. 22** is a perspective view of a disinfectant cap **1010** with inner variable thread segments **1021,** and **FIG. 23** is a side view of the disinfectant cap **1010** of **FIG. 22****.** Although variable thread segments **1021** are discussed with respect to a disinfectant cap **1010,** the variable thread segments **1021** could be used for any threaded medical device (e.g., any threaded luer access device). Further, the variable threads shown could be used with any of the embodiments discussed above.

The disinfectant cap **1010** includes a cap body **1012** with a sidewall **1014** and a bottom wall **1016** defining an interior. The top of the sidewall **1014** defines a top opening **1018.** Although not shown, a removable film could be provided over the top opening **1018.** The outer surface of the sidewall **1014** includes a plurality of annularly spaced vertical ribs **1020** oriented along the axis of the disinfectant cap **1010.** The vertical ribs **1020** facilitate gripping and twisting of the cap **1010** by a user. The inner surface of the sidewall **1014** includes one or more variable thread segments **1021.**

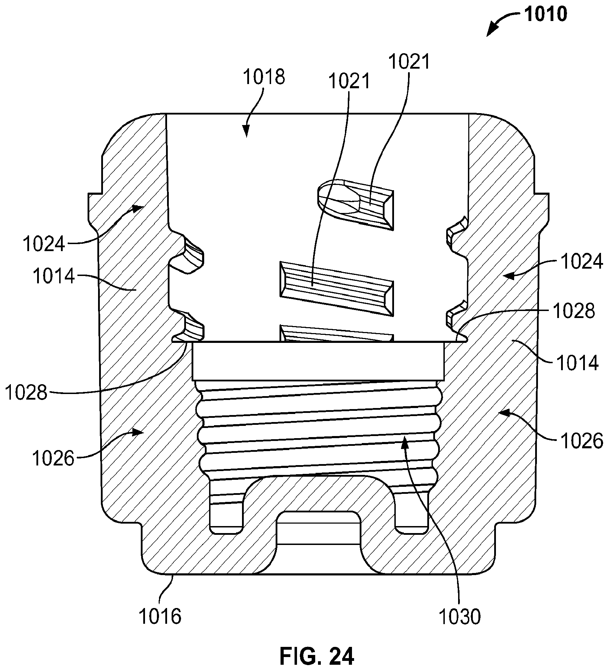

**FIG. 24** is a cross-sectional view of the disinfectant cap **1010** of **FIG. 22****.** The sidewall **1014** includes an upper portion **1024** and a lower portion **1026,** where the sidewall **1014** of the upper portion **1024** is thinner than that of the lower portion **1026** , thereby defining an annular ledge **1028.**

The inner surface of the upper portion **1024** of the disinfectant cap includes variable pitch thread segments **1021** (e.g., helical threads with one or more breaks or interruptions). The variable thread segments **1021** operate similarly to the threads of the disinfectant cap of **FIGS. 19-23****,** such that the variable thread segments **1021** start out at a constant pitch (e.g., starting pitch) and then change pitch thereafter. Further, the breaks between the thread segments **1021** could provide manufacturing advantages. For example, the breaks allow for a corepin tooling designs with slides to release the threads by turning ninety degrees and then pulling out. Additionally, the breaks between the thread segments **1021** allows for the sidewalls **1014** of the disinfectant cap **1010** to more easily expand or stretch if the corepin is pulled directly out during demolding.

The lower portion **1026** of the inner surface of the sidewall **1014** includes retaining rings **1030,** which is discussed in more detail below with respect to **FIGS. 25-26****.**

**FIG. 25** is a top view of the disinfectant cap **1010** of **FIG. 22** with a pre-soaked absorbent material **1032** inserted therein, and **FIG. 26** is a cross-sectional view of the disinfectant cap **1010** of **FIG. 25** with the pre-soaked absorbent material **1032** inserted therein. The lower portion **1026** of the disinfectant cap **1010** could include retaining rings **1030** (e.g., threads, protrusions, etc.) to better retain the absorbent material **1032** (e.g., sponge, pad, cellulos type pad, etc.) therein. As the disinfectant cap **1010** is being assembled, the absorbent material **1032** is positioned within the lower portion **1026** and then wetted and saturated with an antiseptic fluid, so that it expands and wedges itself therein. More specifically, the expanded absorbent material **1032** causes an interlocking effect with the retaining rings **1030,** thereby preventing it from falling out of the disinfectant cap **1010,** such as when the disinfectant cap **1010** is held upside down just before or after removal of the disinfectant cap from the LAD (or other medical device). Alternatively, the absorbent material **1032** could be wetted prior to assembly with the disinfectant cap **1010.** An absorbent material **1032** (e.g., absorbent pad) could be retained within the disinfectant cap **1010** through other methods such as by adhesive, hot-melt, ultrasonically welding, etc.

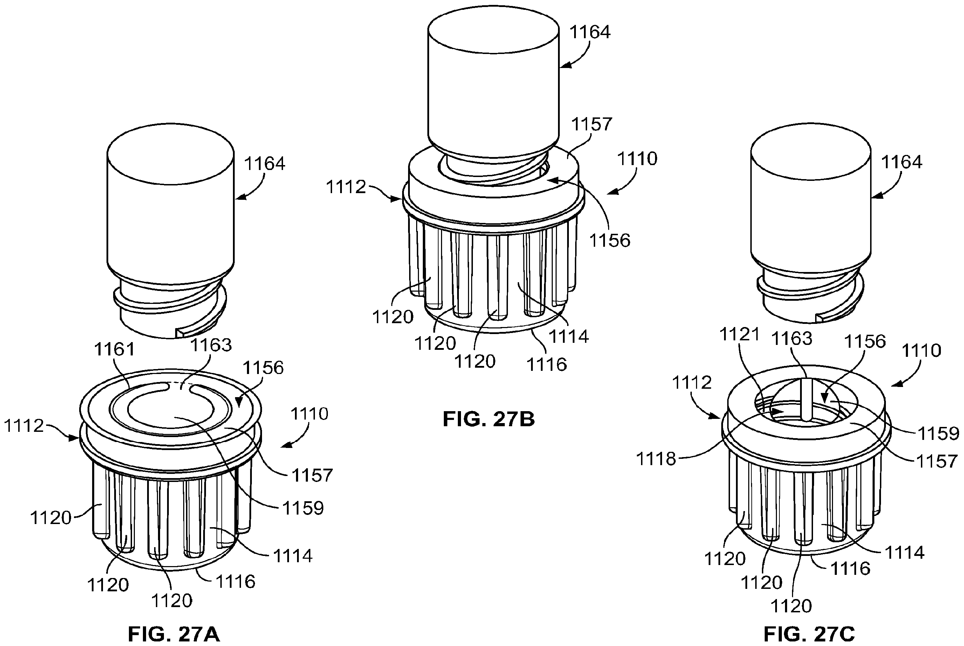

**FIGS. 27A-27C** are perspective views of a disinfectant cap sealed by a film with a scored area. Like other embodiments discussed above (e.g., the embodiment of **FIGS. 19-****26),** the disinfectant cap **1110** includes a cap body **1112** with a sidewall **1114** and a bottom wall **1116** defining an interior. The top of the sidewall **1114** defines a top opening **1118.** The outer surface of the sidewall **1116** could include a plurality of annularly spaced vertical ribs **1120** oriented along the axis of the disinfectant cap **1110.** The inner surface of the sidewall **1114** could include one or more variable threads **1121** (and/or standard threads and/or other attachment means including snaps, interferences, etc.). The disinfectant cap **1110** can be stored in a second component, which can serve as an applicator and/or as packaging. The vertical ribs **1120** can provide rotational lock between the disinfectant cap **1110** and the second component (e.g., applicator). Lidstock can be applied to the second component (e.g., applicator) to provide a fully sterile packaging for the disinfectant cap **1110.**

A film **1156** could be provided over the top opening **1118** to seal antiseptic material (e.g., antiseptic liquid, antiseptic fluid, etc.) within the antiseptic cap **1110.** The film **1156** could have an outer base **1157** and an inner flap **1159,** which are separable from one another by a scored area **1161** but remain connected to one another by an attached area **1163.** The scored area **1161** could be perforated or otherwise weakened (e.g., in a generally circular shape) to facilitate separating the inner flap **1159** from the outer base **1157,** except at the attached area **1163** (e.g., the gap in the generally circular shape of the scored area **1161).** At the attached area **1163,** the inner flap **1159** remains attached to the outer base **1157.** Accordingly, the scored area **1161** is in a "C" shape and is not cut at a full 360 degrees in order to retain a point of attachment between the outer base **1157** and the inner flap **1159** (e.g., the attached area **1163).**

As shown in **FIG. 27A****,** the outer base **1157** and the inner area **1159** are coplanar and connected to one another by the scored area **1161** and the attached area **1163.** In **FIG. 27B****,** the medical implement **1164** (e.g., LAD) is inserted into the antiseptic cap **1110** by puncturing the film **1156.** More specifically, as the medical implement **1164** contacts the film **1156** and is inserted into the top opening **1118** of the disinfectant cap **1110,** the inner flap **1159** separates from the outer base **1157** along the scored area **1161,** and folds downwardly (e.g., into the interior of the disinfectant cap **1110)** about the attached area **1163** (which acts as a hinge). In **FIG. 27C****,** once the medical implement **1164** is disinfected and ready for reuse, the disinfectant cap **1110** is removed from the medical implement **1164,** and the film remains intact (e.g., by the attached area **1163).**

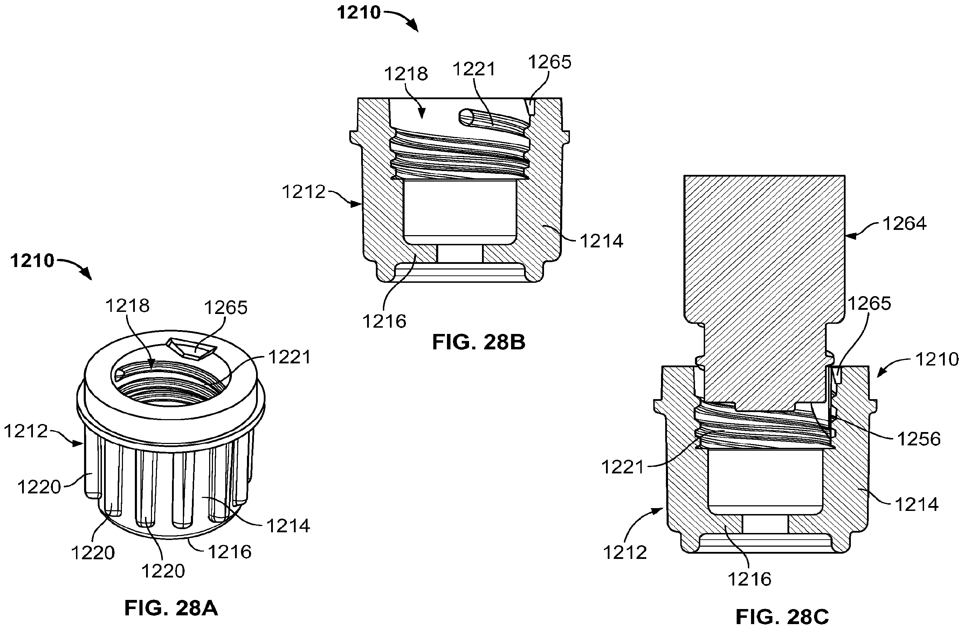

**FIGS. 28A-28C** are perspective views of a disinfectant cap with a notch for receiving an attached area of a film attached to a disinfectant cap. Like the embodiment of **FIGS. 27A-27C** discussed above, the disinfectant cap **1210** includes a cap body **1212** with a sidewall **1214** and a bottom wall **1216** defining an interior. The top of the sidewall **1214** defines a top opening **1218.** The outer surface of the sidewall **1216** could include a plurality of annularly spaced vertical ribs **1220** oriented along the axis of the disinfectant cap **1210.** The inner surface of the sidewall **1214** includes one or more variable threads **1221** (and/or standard threads). The disinfectant cap **1210** can be stored in a second component, which can serve as an applicator and/or as packaging. The vertical ribs **1220** can provide rotational lock between the disinfectant cap **1210** and the second component (e.g., applicator). Lidstock can be applied to the second component (e.g., applicator) to provide a fully sterile packaging for the disinfectant cap **1210.**

A film **1256** could be provided over the top opening **1218,** to seal antiseptic material (e.g., antiseptic liquid, antiseptic fluid, etc.) within the antiseptic cap **1210,** as discussed in **FIGS. 27A-27C****.** The film **1256** could have an outer base, an inner flap, scored area, and attached area, as discussed above. However, the scored area is optional and not required.

As shown in **FIG. 28A-28B****,** the disinfectant cap **1210** could include a notch **1265** (or a plurality of notches) in an interior surface at the rim (e.g., at the top opening **1218)** of the disinfectant cap **1210.** The notch **1265** aligns with the attached area of the film **1256.** In this way, as shown in **FIG. 28C****,** when the medical device **1264** (e.g., LAD) engages the disinfectant cap **1210,** the attached area folds into the notch **1265** when the inner flap separates from the outer base and folds downwardly (e.g., into the interior of the disinfectant cap **1210)** about the attached area. This relieves friction and pressure on the attached area as the medical implement **1264** engages the disinfectant cap **1210,** the attached area remains intact (and that the inner flap remains attached to the outer base at the attached area). The notch **1265** also provides a break in the edge of the cap **1210,** along which a shearing force is created on the film (e.g., lidstock) as the LAD is inserted into the cap **1210.** This break causes the film to shear around the entire circumference of the film, except at the notch **1265,** thus creating the attached area and preventing the film from fully separating from the cap **1210.**

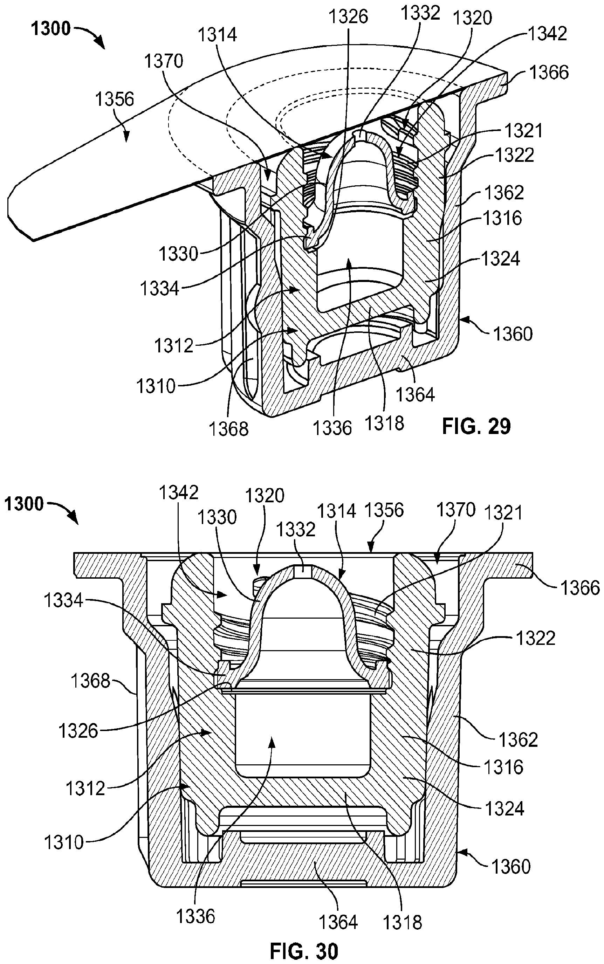

**FIGS. 29-30** are views of a disinfectant cap with a dome insert sealed in a cap holder. More specifically, **FIG. 29** is a cross-sectional perspective view of a disinfectant cap with a dome insert sealed in a cap holder, and **FIG. 30** is a cross-sectional side view of the disinfectant cap and cap holder of **FIG. 29****.** Like the other embodiments discussed above (e.g., the embodiments of **FIGS. 13-15****),** the disinfectant cap **1310** includes an elastomeric dome insert **1314** positioned within the interior of the cap body **1312.** The elastomeric dome insert **1314** includes a dome **1330** (e.g., hemispherical wall) having a hole **1332** (e.g., slit, opening, and/or weakened area) formed through (or substantially near) the apex thereof. The size of the hole **1332** can be sized to minimize the amount of disinfectant that might leak therethrough if the cap **1320** is positioned upside down prior to insertion of the medical device (e.g., LAD). For example, a small hole may be prone to contain the disinfectant as a liquid tends not to flow through a small hole unless pressurized (e.g., due to liquid surface tension). The elastomeric dome insert **1314** further includes an annular disc **1334** extending outwardly from the base of the dome **1330.**

The disinfectant cap **1310** includes a cap body **1312** with a sidewall **1316** and a bottom wall **1318** defining an interior. The top of the sidewall **1316** defines a top opening **1320.** The sidewall **1316** includes an upper portion **1322** and a lower portion **1324,** with a shoulder (e.g., ledge) **1326** therebetween, such that the wall of the upper portion **1322** has a smaller thickness than the wall of the lower portion **1324.** The annular disc **1334** rests on the shoulder (e.g., ledge) **1326** of the cap body **1312** when positioned therein. The lower portion **1324** of the sidewall **1316** of the cap body **1312** and the bottom surface of the elastomeric dome insert **1314** define a first chamber **1336** for containing antiseptic fluid. The upper portion **1322** of the sidewall **1316** of the cap body **1312** and the top surface of the elastomeric dome insert **1314** define a second chamber **1342** for receiving and engaging a medical device (e.g., LAD). The inner surface of the upper portion **1322** of the sidewall **1316** could include threads **1321** (e.g., variable and/or standard threads) and/or be elastically deformable to engage the LAD.

The disinfectant cap assembly **1300** includes the disinfectant cap **1310,** elastomeric dome insert **1314,** and cap holder **1360.** The cap holder **1360** includes a sidewall **1362** and a bottom wall **1364** defining an interior. The top of the sidewall **1362** defines a top opening **1370** and includes an outwardly extending flange **1366.** The outer surface of the sidewall **1316** could include a plurality of annularly spaced vertical ribs **1368** oriented along the axis of the cap holder **1360.** A lidstock **1356** could be attached to the flange **1366** of the cap holder **1360** to seal the disinfectant cap **1310** therein. The top of the sidewall **1316** of the disinfectant cap **1310** could also be attached to the lidstock **1356** to seal the elastomeric dome insert **1314** and antiseptic fluid within the disinfectant cap **1310.**

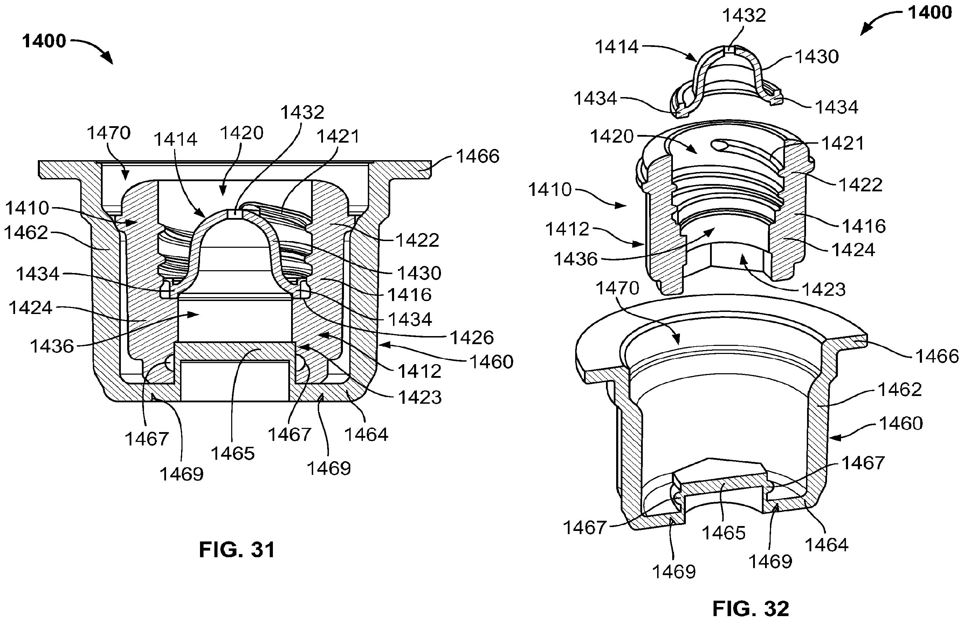

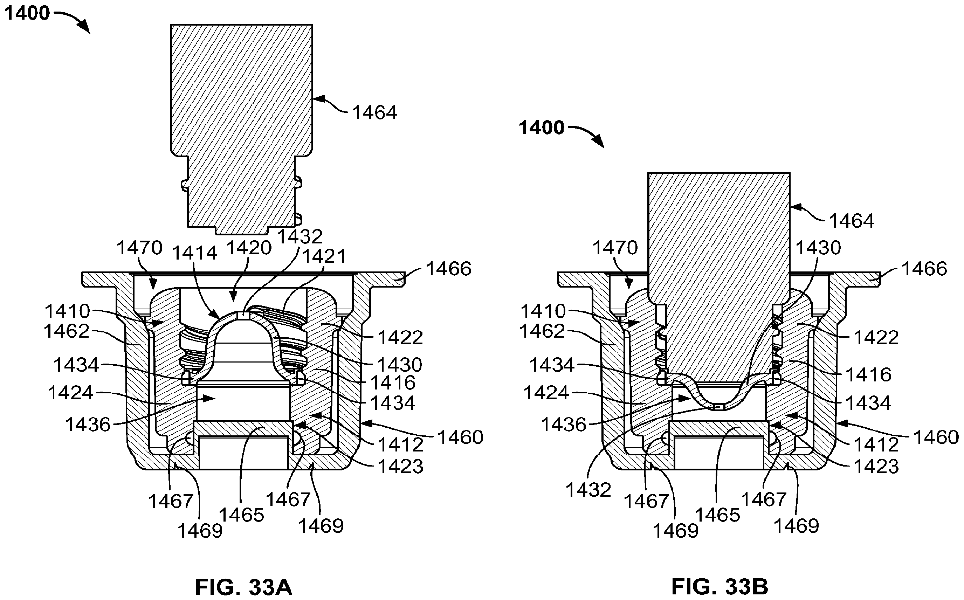

**FIGS. 31-33B** are views of a disinfectant cap with a dome insert sealed in a cap holder, the disinfectant cap having a bottom opening. More specifically, **FIG. 31** is a cross-sectional side view of a disinfectant cap with a dome insert in a cap holder, the disinfectant cap having a bottom opening, **FIG. 32** is an exploded cross-sectional perspective view of the disinfectant cap and cap holder of **FIG. 31****,** and **FIGS. 33A****-33B** are cross-sectional side views illustrating use of the disinfectant cap and cap holder of **FIG. 31****.** Like the other embodiments discussed above (e.g., the embodiments of **FIGS. 13-15** and **29-30),** the disinfectant cap **1410** includes an elastomeric dome insert **1414** having a dome **1430** with a hole **1432.** The elastomeric dome insert **1414** further includes an annular disc **1434** extending outwardly from the base of the dome **1430.**

The disinfectant cap **1410** includes a cap body **1412** with a sidewall **1416** defining an interior. The top of the sidewall **1416** defines a top opening **1420,** and the bottom of the sidewall **1416** defines a bottom opening **1423.** The bottom opening **1423** could be hexagonally shaped (or otherwise shaped, such as circularly shaped). The sidewall **1416** includes an upper portion **1422** and a lower portion **1424,** with a shoulder **1426** therebetween. The disinfectant cap assembly **1400** includes the disinfectant cap **1410,** elastomeric dome insert **1414,** and cap holder **1460.** The cap holder **1460** includes a cap holder body **1461** with a sidewall **1462** and a bottom wall **1464** defining an interior. The top of the sidewall **1462** defines a top opening **1470** and includes an outwardly extending flange **1466.** The outer surface of the sidewall **1416** could include a plurality of annularly spaced vertical ribs **1468** oriented along the axis of the cap holder **1460.** As described above, a lidstock (not shown) could be attached to the flange **1466** of the cap holder **1460** to seal the disinfectant cap **1410** therein. The top of the sidewall **1416** of the disinfectant cap **1410** could also be attached to the lidstock to seal the elastomeric dome insert **1414** and antiseptic fluid within the disinfectant cap **1412.**

The cap holder **1470** could further include an inwardly protruding base **1465** extending from an approximate center of the bottom wall **1464** of the cap holder **1460.** The base **1465** includes one or more nubs **1467** extending from a perimeter of the base **1465.** The base **1465** is approximately sized to that of the bottom opening **1423** of the disinfectant cap **1410** to be received therein. The sides of the base **1465** and/or the one or more nubs **1467** frictionally engage and/or partially deform the walls forming the bottom opening **1423** to secure the disinfectant cap **1410** to the cap holder **1460.**

The lower portion **1424** of the sidewall **1416** of the cap body **1412,** the bottom surface of the elastomeric dome insert **1414,** and the top surface of the cap holder base **1465** define a first chamber **1436** for containing antiseptic fluid. The upper portion **1422** of the sidewall **1416** of the cap body **1412** and the top surface of the elastomeric dome insert **1414** define a second chamber **1442** for receiving and engaging a medical device (e.g., LAD). The inner surface of the upper portion **1422** of the sidewall **1416** could include threads **1421** (e.g., variable and/or standard threads) and/or be elastically deformable to engage the LAD.

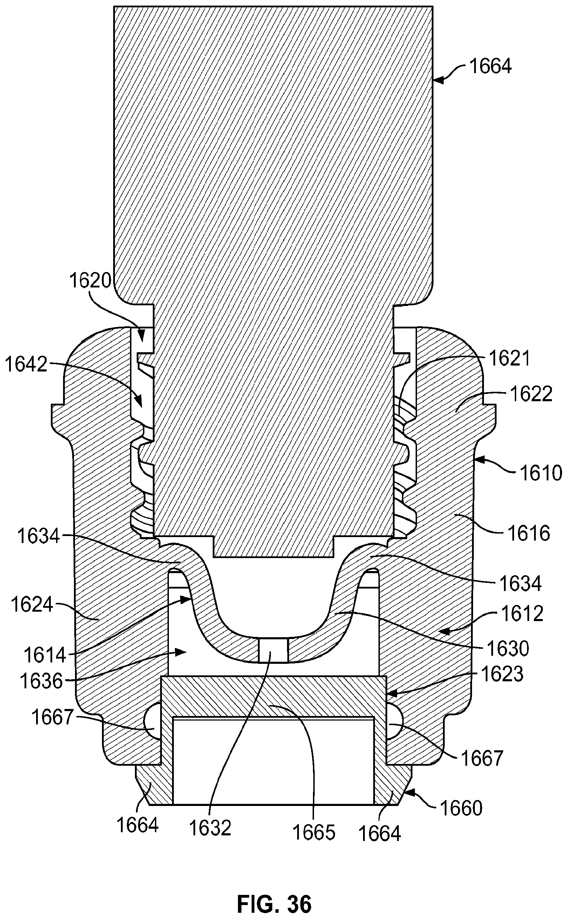

Further, the bottom wall **1464** could include one or more annular cuts **1469** (e.g., or thin webbing, or other weakened area) therein generally surrounding the inwardly protruding base **1465.** Accordingly, once a user fully threads the cap **1410** and cap holder **1460** onto a medical device, the user can then continue to twist, and break away the inwardly protruding base **1465** from the rest of the cap holder **1460** along the one or more cuts **1469.** This leaves the inwardly protruding base **1465** still fully engaged with the cap **1410,** like the cap shown in **FIG. 36** below, where the cap **1410** remains engaged with the medical device. The cap **1410** can then be unscrewed to use the medical device.

As shown in **FIGS. 33A-33B****,** antiseptic fluid is retained within the first chamber **1436.** As the disinfectant cap **1410** and cap holder **1460** engage the medical device **1464,** the elastically deformable insert **1414** inwardly deforms increasing pressure of the antiseptic fluid (e.g., by decreasing the volume of first chamber **1436),** and antiseptic fluid then flows onto the medical device **1464.**

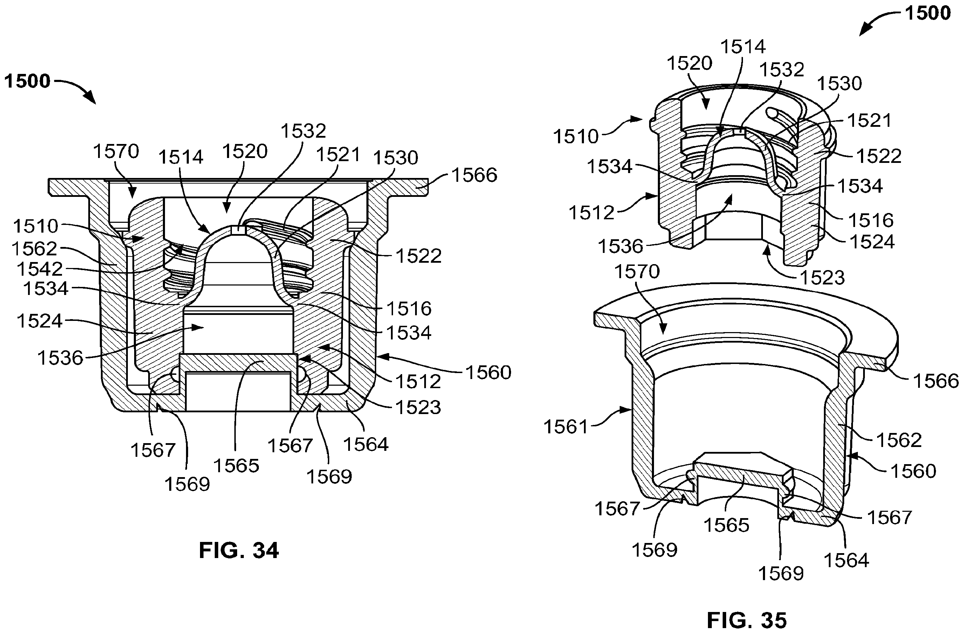

**FIGS. 34-35** are views of a disinfectant cap with an integrally formed internal dome barrier in a cap holder, the disinfectant cap having a bottom opening. More specifically, **FIG. 34** is a cross-sectional side view of a disinfectant cap with an integrally formed internal dome barrier in a cap holder, the disinfectant cap having a bottom opening, and **FIG. 35** is an exploded cross-sectional perspective view of the disinfectant cap and cap holder of **FIG. 34****.**

The embodiment of **FIGS. 34-35** is like that of **FIGS. 31-33B****.** The disinfectant cap **1510** includes a cap body **1512** with a sidewall **1516** defining an interior. The top of the sidewall **1516** defines a top opening **1520,** and the bottom of the sidewall **1516** defines a bottom opening **1523** (e.g., hexagonally shaped). The sidewall **1516** includes an upper portion **1522** and a lower portion **1524.** The inner surface of the upper portion **1522** of the sidewall **1516** could include threads **1521** (e.g., variable and/or standard threads) and/or be elastically deformable to engage the LAD.

The disinfectant cap assembly **1500** includes the disinfectant cap **1510** and cap holder **1560.** The cap holder **1560** includes a cap holder body **1561** with a sidewall **1562** and a bottom wall **1564** defining an interior. The top of the sidewall **1562** defines a top opening **1570** and includes an outwardly extending flange **1566.** The outer surface of the sidewall **1516** could include a plurality of annularly spaced vertical ribs (not shown). As described above, a lidstock (not shown) could be attached to the flange **1566** of the cap holder **1560** to seal the disinfectant cap **1510** therein. The top of the sidewall **1516** of the disinfectant cap **1510** could also be attached to the lidstock to seal the elastomeric dome barrier **1514** and antiseptic fluid within the disinfectant cap **1512.** The cap holder **1570** could further include an inwardly protruding base **1565** extending from an approximate center of the bottom wall **1564** of the cap holder **1560.** The base **1565** includes one or more nubs **1567** extending from a perimeter of the base **1565.**

In this embodiment, the disinfectant cap **1510** includes an elastomeric dome barrier **1514** integrally formed with and attached to the body **1512** of the disinfectant cap **1510.** The elastomeric dome barrier **1514** has a dome **1530** with a hole **1532** at (or proximate to) the apex thereof. The base **1534** of the elastomeric dome barrier **1514** is integrally attached to the interior surface of the sidewalls **1516** of the disinfectant cap **1510.** Accordingly, the lower portion **1524** of the sidewall **1516** of the cap body **1512,** the bottom surface of the elastomeric dome barrier **1514,** and the top surface of the cap holder base **1565** define a first chamber **1536** for containing antiseptic fluid. The upper portion **1522** of the sidewall **1516** of the cap body **1512** and the top surface of the elastomeric dome barrier **1514** define a second chamber **1542** for receiving and engaging a medical device (e.g., LAD).

Further, the bottom wall **1564** could include one or more annular cuts **1569** (e.g., or thin webbing, or other weakened area) therein generally surrounding the inwardly protruding base **1565.** Accordingly, once a user fully threads the cap **1510** and cap holder **1560** onto a medical device, the user can then continue to twist, and break away the inwardly protruding base **1565** from the rest of the cap holder **1560** along the one or more cuts **1569.** This leaves the inwardly protruding base **1565** still fully engaged with the cap **1510,** like the cap shown in **FIG. 36** below, where the cap **1510** remains engaged with the medical device. The cap **1510** can then be unscrewed to use the medical device.

Alternately, the first chamber **1536** could be formed by the bottom surface of the dome barrier **1514,** the sidewall **1516** of the cap body **1512,** and an additional piece (not shown) that is inserted into the opening **1523.** This allows the formation of the first chamber **1536** without using a surface of the cap holder **1560.** This also allows the entire cap **1510** to set within the sterile packaging formed by the cap holder **1560** and the lidstock.

**FIG. 36** is a cross-sectional view of a disinfectant cap with a bottom plug. The disinfectant cap of **FIG. 36** is like the disinfectant cap of **FIGS. 34-35****.** The disinfectant cap **1610** includes a cap body **1612** with a sidewall **1616** defining an interior. The top of the sidewall **1616** defines a top opening **1620,** and the bottom of the sidewall **1616** defines a bottom opening **1623** (e.g., hexagonally shaped). The sidewall **1616** includes an upper portion **1622** and a lower portion **1624.** The inner surface of the upper portion **1622** of the sidewall **1616** could include threads **1621** (e.g., variable and/or standard threads) and/or be elastically deformable to engage the LAD. The disinfectant cap **1610** includes an elastomeric dome barrier **1614** integrally formed with and attached to the body **1612** of the disinfectant cap **1610.** The elastomeric dome barrier **1614** has a dome **1630** with a hole **1632** at (or proximate to) the apex thereof. The base **1634** of the elastomeric dome barrier **1614** is integrally attached to the interior surface of the sidewalls **1616** of the disinfectant cap **1610.** As described above, a lidstock (not shown) could be attached to the disinfectant cap **1610.**

The disinfectant cap **1610** includes a plug **1660.** The plug **1660** includes an inwardly protruding base **1665** and outwardly extending flanges **1667.** The base **1665** is approximately sized to that of the bottom opening **1623** of the disinfectant cap **1610** to be received therein. The sides of the base **1665** and/or the one or more nubs **1667** frictionally engage and/or partially deform the walls forming the bottom opening **1623** to secure the plug **1660** to the disinfectant cap **1610.** The flanges **1667** prevent over insertion of the plug **1660** into the disinfectant cap **1610.** Accordingly, the lower portion **1624** of the sidewall **1616** of the cap body **1612,** the bottom surface of the elastomeric dome barrier **1614,** and the top surface of the plug **1660** define a first chamber **1636** for containing antiseptic fluid. The upper portion **1622** of the sidewall **1616** of the cap body **1612** and the top surface of the elastomeric dome barrier **1614** define a second chamber **1642** for receiving and engaging a medical device **1664** (e.g., LAD).

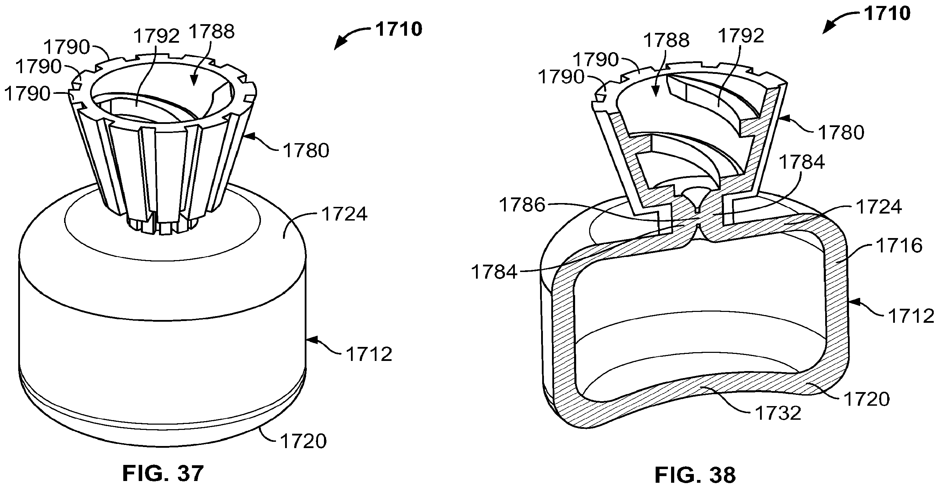

**FIGS. 37-38** are views of a disinfectant cap with a frangible neck. More specifically, **FIG. 37** is a perspective view of a disinfectant cap with a frangible neck, and **FIG. 38** is a cross-sectional perspective view of the disinfectant cap of **FIG. 37****.** The disinfectant cap **1710** includes a cap body **1712** with a sidewall **1716,** a bottom wall **1720,** and top wall **1724** defining an interior. The bottom wall **1720** of the cap body **1712** can include an invertible wall **1732** (as described in more detail above).

Extending from the top wall of the disinfectant cap is a neck **1784** which has been crimped (e.g., heat crimped) closed to form a frangible stop **1786.** Extending from the neck **1784** is a receptacle **1780** defining a top opening **1788** and conical interior (e.g., tapered interior) to engage a medical device (e.g., LAD). The receptacle **1780** is of a generally inverted cone such that the top of the cone **1780** is wider than the base (e.g., where the receptacle **1780** attaches to the neck **1784).** The inner surface of the receptacle **1780** includes one or more threads **1792** (variable and/or standard threads). The exterior surface of the receptacle **1780** could include a plurality of annularly spaced vertical ribs **1790.** In this way, the antiseptic cap **1710** could be a one piece construction (e.g., all of the components are integrally connected to one another). A lidstock (not shown) could be attached to the top opening **1788** of the receptacle **1780.**

As the medical device is inserted (e.g., threadably inserted) into the receptacle **1780,** the taper of the receptacle **1780** forces the head **1780** apart (e.g., the narrowest point of the conical head **1780** closest to the neck **1784** is widened). As a result, the frangible stop **1786** is eventually forced open, and the antiseptic fluid contained within the body **1712** of the disinfectant cap **1710** flows out of the disinfectant cap **1710** through the neck **1786.**

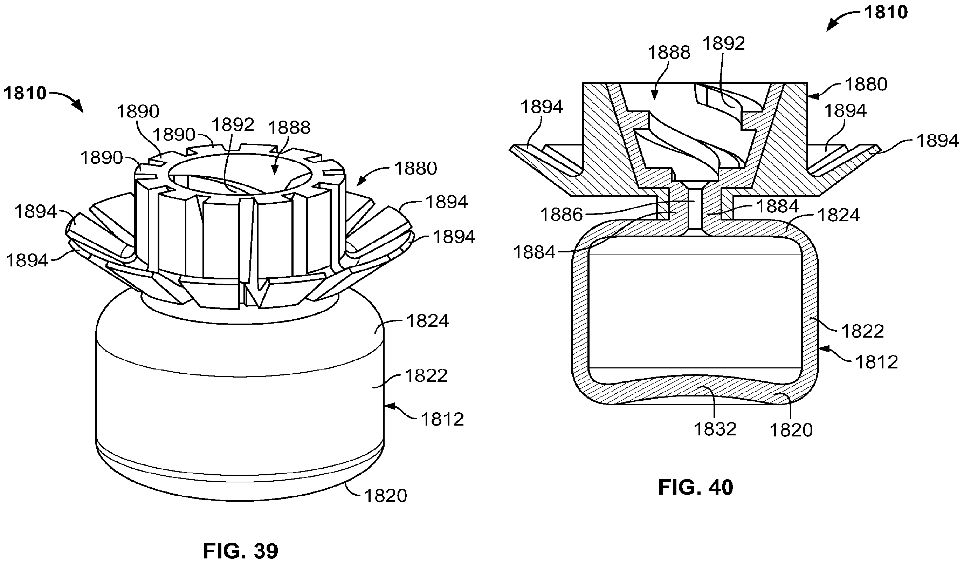

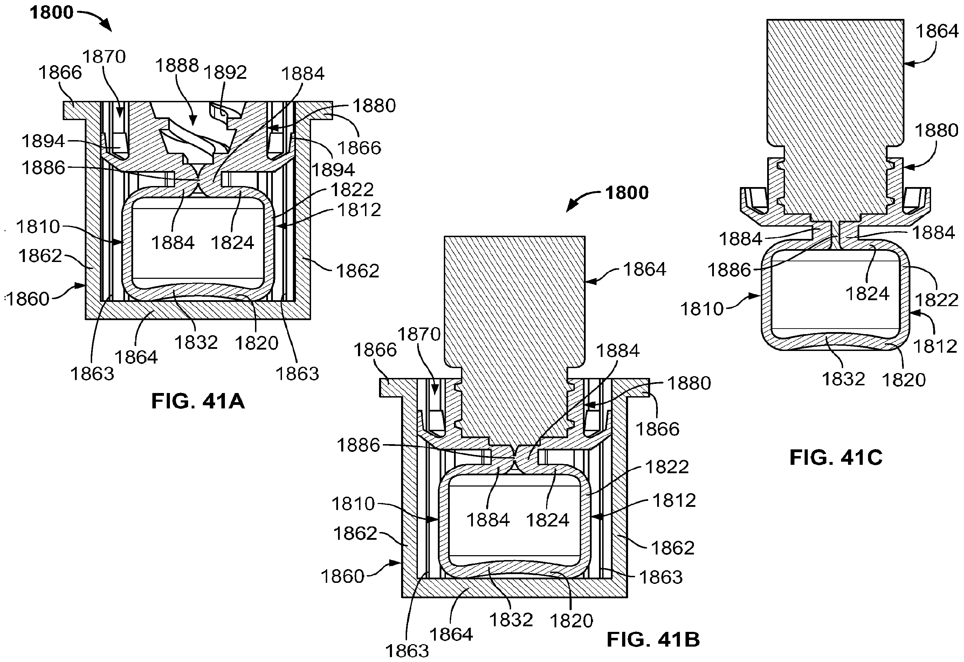

**FIGS. 39-41C** are views of a disinfectant cap with a frangible neck and angled fingers. More specifically, **FIG. 39** is a perspective view of a disinfectant cap with a frangible neck and angled fingers, **FIG. 40** is a cross-sectional perspective view of the disinfectant cap of **FIG. 39****,** and **FIGS. 41A****-41C** are cross-sectional side views illustrating use of the disinfectant cap of **FIG. 39** and cap holder. The disinfectant cap of **FIGS. 39-****41C** is like the disinfectant cap of **FIGS. 37-38****.**

The disinfectant cap **1810** includes a cap body **1812** with a sidewall **1816,** a bottom wall **1820,** and top wall **1824** defining an interior. The bottom wall **1820** of the cap body **1812** can include an invertible wall **1832** (as described in more detail above). Extending from the top wall of the disinfectant cap is a neck **1884** which forms a channel **1886** prior to being crimped close to form a frangible stop. Extending from the neck **1884** is a receptacle **1880** defining a top opening **1888** and generally conical interior to engage a medical device (e.g., LAD). The conical interior is of a generally inverted cone such that the top of the cone **1880** is wider than the base (e.g., where the receptacle **1880** attaches to the neck **1884).** The inner surface of the receptacle **1880** includes one or more threads **1892** (variable and/or standard threads). The exterior surface of the receptacle **1880** could include a plurality of annularly spaced vertical ribs **1890.** In this way, the antiseptic cap **1810** could be a one piece construction (e.g., all of the components are integrally connected to one another). The receptacle **1880** further includes one or more annularly spaced upwardly angled fingers **1894** extending from a base of the receptacle **1880.**

As shown in **FIG. 41A****,** the disinfectant cap **1810** could be used with a cap holder **1860.** More specifically, the disinfectant cap assembly **1800** includes the disinfectant cap **1810** and cap holder **1860.** The cap holder **1860** includes a sidewall **1862** and a bottom wall **1864** defining an interior. The top of the sidewall **1862** defines a top opening **1870** and includes an outwardly extending flange **1866.** A lidstock (not shown) could be attached to the flange **1866** of the cap holder **1860** to seal the disinfectant cap **1810** therein. The top of the receptacle **1880** of the disinfectant cap **1810** could also be attached to the lidstock. The outer surface of the sidewall **1816** could include a plurality of annularly spaced vertical ribs (not shown) oriented along the axis of the cap holder **1860.** The interior surface of the sidewall **1816** could include a plurality of annularly spaced vertical ribs **1863.**

As shown, the outermost diameter formed by the angled fingers **1894** could be greater than the inner diameter of the cap holder **1860.** As a result, radial forces of the angled fingers **1894** against the cap holder keep the neck **1884** closed (thereby retaining the antiseptic fluid therein). Further, the width of the angled fingers **1894** could be approximately the same width as the spacing between the plurality of vertical ribs **1863.**

As shown in **FIG. 41B****,** as the medical device **1864** is inserted (e.g., threadably inserted) into the receptacle **1880,** the base of the head **1880** (e.g., the narrowest point of the conical head **1880** closest to the neck **1884),** cannot forces the head **1880** apart while it is retained within the cap holder **1860.** The vertical ribs **1863** of the cap holder **1860** interact with the fingers **1894** of the disinfectant cap **1810** to facilitate threading of the disinfectant cap **1810** onto the medical device **1864** by preventing relative rotation of the disinfectant cap **1810** and cap holder **1860.**

As shown in **FIG. 41C****,** once the disinfectant cap **1810** is fully engaged with the medical device **1864,** the cap holder **1860** is removed from the disinfectant cap **1810.** At this point, the frangible stop **1886** is forced open and forms a channel, and the antiseptic fluid contained within the body **1812** of the disinfectant cap **1810** flows out of the disinfectant cap **1810** through the neck **1886** and onto the medical device **1864.**

A number of the components and features discussed above could be integrally constructed or separately attached. For example, the disinfectant cap and cover of **FIGS. 1-3** could be made of a single piece (e.g., the tapered portion being integrally formed with or as part of the outer walls of the disinfectant cap).

Having thus described the system and method in detail, it is to be understood that the foregoing description is not intended to limit the spirit or scope thereof. It will be understood that the embodiments of the present disclosure described herein are merely exemplary and that a person skilled in the art may make any variations and modification without departing from the spirit and scope of the disclosure. All such variations and modifications, including those discussed above, are intended to be included within the scope of the disclosure.

## Claims

1. A disinfectant cap for disinfecting a surface of a medical device, comprising:
a cap body (612) having a bottom wall and a sidewall (616) extending therefrom, the sidewall defining a top opening;
antiseptic fluid contained within the cap body; and
a deformable dome insert (614) positioned within the cap body, the dome insert and the cap body defining a first chamber (636) for containing the antiseptic fluid, the dome insert being inwardly deformable from a first position sealing the antiseptic fluid within the cap body to a second position releasing the antiseptic fluid from the cap body.

2. The disinfectant cap of Claim 1, wherein the dome insert (614) is inwardly deformable from a first convex orientation to a second concave orientation.

3. The disinfectant cap of Claim 2, wherein the dome insert (614) is integrally formed with the cap body.

4. The disinfectant cap of any of Claims 1 to 3, further comprising a removable film attached to a top surface of the cap body.

5. The disinfectant cap of any of Claims 1 to 4, wherein the dome insert (614) comprises a partial slit (632) formed therethrough, the partial slit closed when the dome insert is in the first position and opened when the dome insert is inverted to the second position.

6. The disinfectant cap of any of Claims 1 to 5, wherein the dome insert is attached to a top of the cap body.

7. The disinfectant cap of any of Claims 1 to 7, wherein the dome insert is recessed within the cap body.

8. The disinfectant cap of any of Claims 1 to 7, wherein the sidewall includes a lower portion (642) and an upper portion (622) with a shoulder (626) therebetween, the lower portion defining a first chamber (636) for containing the antiseptic fluid, the upper portion defining a second chamber (642) for receiving and engaging the medical device, and the dome insert proximate the shoulder.

9. The disinfectant cap of any of Claims 1 to 8, wherein the medical device is a luer access device.

10. The disinfectant cap of any of Claims 1 to 9, wherein the antiseptic fluid comprises alcohol.

11. The disinfectant cap of any of Claims 1 to 10, wherein the cap body further comprises internal threads.

12. An assembly for disinfecting a surface of a medical device, the assembly comprising:
a cap holder (1360; 1460; 1560) comprising a sidewall and a bottom wall defining an interior, the cap holder being configured to receive the disinfectant cap of any of Claims 1 to 11;
the disinfectant cap of any of Claims 1 to 11; and
a lidstock (1356) being removably attached to a top surface of the cap holder and being configured to seal the disinfectant cap within the cap holder.

13. The assembly of Claim 12, wherein the cap holder further comprises a plurality of vertical ribs along the sidewall.

## Patentansprüche

1. Desinfektionskappe zum Desinfizieren einer Oberfläche einer medizinischen Vorrichtung, die Folgendes beinhaltet:
einen Kappenkörper (612) mit einer Bodenwand und einer sich davon erstreckenden Seitenwand (616), wobei die Seitenwand eine obere Öffnung definiert;
antiseptisches Fluid, das in dem Kappenkörper enthalten ist; und
einen verformbaren kuppelförmigen Einsatz (614), der in dem Kappenkörper positioniert ist, wobei der kuppelförmige Einsatz und der Kappenkörper eine erste Kammer (636) zum Enthalten des antiseptischen Fluids definiert, wobei der kuppelförmige Einsatz von einer ersten Stellung, die das antiseptische Fluid dicht im Kappenkörper einschließt, auf eine zweite Stellung, die das antiseptische Fluid aus dem Kappenkörper freisetzt, einwärts verformbar ist.

2. Desinfektionskappe nach Anspruch 1, wobei der kuppelförmige Einsatz (614) einwärts von einer ersten, konvexen Ausrichtung auf eine zweite, konkave Ausrichtung verformbar ist.

3. Desinfektionskappe nach Anspruch 2, wobei der kuppelförmige Einsatz (614) an den Kappenkörper angeformt ist.

4. Desinfektionskappe nach einem der Ansprüche 1 bis 3, die ferner eine entfernbare Folie aufweist, die an einer oberen Oberfläche des Kappenkörpers angebracht ist.

5. Desinfektionskappe nach einem der Ansprüche 1 bis 4, wobei der kuppelförmige Einsatz (614) einen teilweisen durch ihn hindurch gebildeten Schlitz (632) aufweist, wobei der teilweise Schlitz geschlossen ist, wenn der kuppelförmige Einsatz in der ersten Stellung ist, und geöffnet ist, wenn der kuppelförmige Einsatz in die zweite Stellung umgekehrt wurde.

6. Desinfektionskappe nach einem der Ansprüche 1 bis 5, wobei der kuppelförmige Einsatz an einem oberen Ende des Kappenkörpers angebracht ist.

7. Desinfektionskappe nach einem der Ansprüche 1 bis 7, wobei der kuppelförmige Einsatz im Kappenkörper ausgespart ist.

8. Desinfektionskappe nach einem der Ansprüche 1 bis 7, wobei die Seitenwand einen unteren Teil (642) und einen oberen Teil (622) mit einer Schulter (626) dazwischen, wobei der untere Teil eine erste Kammer (636) zum Enthalten des antiseptischen Fluids definiert, der obere Teil eine zweite Kammer (642) zum Aufnehmen und zum Ineingriffkommen mit der medizinischen Vorrichtung definiert, und den kuppelförmigen Einsatz nahe der Schulter beinhaltet.

9. Desinfektionskappe nach einem der Ansprüche 1 bis 8, wobei die medizinische Vorrichtung eine Luer-Zugangsvorrichtung ist.

10. Desinfektionskappe nach einem der Ansprüche 1 bis 9, wobei das antiseptische Fluid Alkohol aufweist.

11. Desinfektionskappe nach einem der Ansprüche 1 bis 10, wobei der Kappenkörper ferner ein Innengewinde aufweist.

12. Anordnung zum Desinfizieren einer Oberfläche einer medizinischen Vorrichtung, wobei die Anordnung Folgendes aufweist:
einen Kappenhalter (1360; 1460; 1560), der eine Seitenwand und eine Bodenwand aufweist, die ein Inneres definieren, wobei der Kappenhalter zum Aufnehmen der Desinfektionskappe nach einem der Ansprüche 1 bis 11 gestaltet ist;
die Desinfektionskappe nach einem der Ansprüche 1 bis 11; und
ein Deckelmaterial (1356), das abnehmbar an einer oberen Oberfläche des Kappenhalters angebracht ist und zum dichten Einschließen der Desinfektionskappe in dem Kappenhalter gestaltet ist.

13. Anordnung nach Anspruch 12, wobei die Kappenhalter ferner eine Vielzahl von vertikalen Rippen an der Seitenwand entlang aufweist.

## Revendications

1. Capuchon désinfectant pour désinfecter une surface d'un dispositif médical, comprenant
un corps de capuchon (612) présentant une paroi inférieure et une paroi latérale (616) s'étendant à partir de celle-ci, la paroi latérale définissant une ouverture supérieure ;
un fluide antiseptique contenu à l'intérieur du corps de capuchon ; et
un insert bombé déformable (614) positionné à l'intérieur du corps de capuchon, l'insert bombé et le corps de capuchon définissant une première chambre (636) destinée à contenir le fluide antiseptique, l'insert bombé étant déformable vers l'intérieur d'une première position qui scelle le fluide antiseptique à l'intérieur du corps de capuchon à une seconde position qui libère le fluide antiseptique du corps de capuchon.

2. Capuchon désinfectant selon la revendication 1, dans lequel l'insert bombé (614) est déformable vers l'intérieur d'une première orientation convexe à une seconde orientation concave.

3. Capuchon désinfectant selon la revendication 2, dans lequel l'insert bombé (614) fait partie intégrante du corps de capuchon.

4. Capuchon désinfectant selon l'une quelconque des revendications 1 à 3, comprenant en outre une pellicule amovible attachée à une surface supérieure du corps de capuchon.

5. Capuchon désinfectant selon l'une quelconque des revendications 1 à 4, dans lequel l'insert bombé (614) comprend une fente partielle (632) formée à travers celui-ci, la fente partielle étant fermée quand l'insert bombé est dans la première position et ouverte quand l'insert bombé est inversé sur la seconde position.

6. Capuchon désinfectant selon l'une quelconque des revendications 1 à 5, dans lequel l'insert bombé est attaché à une partie supérieure du corps de capuchon.

7. Capuchon désinfectant selon l'une quelconque des revendications 1 à 7, dans lequel l'insert bombé est renfoncé à l'intérieur du corps de capuchon.

8. Capuchon désinfectant selon l'une quelconque des revendications 1 à 7, dans lequel la paroi latérale comporte une partie inférieure (642) et une partie supérieure (622) avec un épaulement (626) entre elles, la partie inférieure définissant une première chambre (636) destinée à contenir le fluide antiseptique, la partie supérieure définissant une seconde chambre (642) destinée à recevoir et à s'enclencher avec le dispositif médical, et l'insert bombé proche de l'épaulement.

9. Capuchon désinfectant selon l'une quelconque des revendications 1 à 8, dans lequel le dispositif médical est un dispositif d'accès Luer.

10. Capuchon désinfectant selon l'une quelconque des revendications 1 à 9, dans lequel le fluide antiseptique comprend de l'alcool.

11. Capuchon désinfectant selon l'une quelconque des revendications 1 à 10, dans lequel le corps de capuchon comprend en outre des filets internes.

12. Ensemble de désinfection d'une surface d'un dispositif médical, l'ensemble comprenant :
un porte-capuchon (1360 ; 1460 ; 1560) comprenant une paroi latérale et une paroi inférieure définissant un intérieur, le porte-capuchon étant configuré pour recevoir le capuchon désinfectant selon l'une quelconque des revendications 1 à 11;
le capuchon désinfectant selon l'une quelconque des revendications 1 à 11 ; et
un matériau d'operculage (1356) fixé de manière amovible à une surface supérieure du porte-capuchon et configuré pour sceller le capuchon désinfectant à l'intérieur du porte-capuchon.

13. Ensemble selon la revendication 12, dans lequel le porte-capuchon comprend en outre une pluralité de nervures verticales le long de la paroi latérale.
